# EUROPEAN PATENT APPLICATION

(11) **EP 4 137 161 A1**
(43) Date of publication of application: **22.02.2023**
(21) Application number: 21787537.6
(22) Date of filing: 15.04.2021
(51) Int. Cl.: A61K 48/00, C12N 15/113, C12N 15/11, C12N 15/88, A61P 3/00

(54) **METHOD AND DRUG FOR TREATING HURLER SYNDROME**

(30) Priority: 15.04.2020 WO PCT/CN2020/084925
(71) Applicant: EdiGene Therapeutics (Beijing) Inc., Beijing 102206 (CN)
(72) Inventor: YUAN, Pengfei, Beijing 102206 (CN); ZHAO, Yanxia, Beijing 102206 (CN); LIU, Nengyin, Beijing 102206 (CN); YI, Zexuan, Beijing 102206 (CN); TANG, Gangbin, Beijing 102206 (CN)
(74) Representative: Lavoix
(86) International application number: PCT/CN2021/087534
(87) International publication number: WO 2021/209010

(57) **Abstract**

Disclosed are a LEAPER technique-based nucleic acid drug and a method for treating diseases such as Hurler syndrome by means of using the nucleic acid drug to target and edit RNA. The method comprises: performing an editing from adenosine base to hypoxanthine base on RNA by using the nucleic acid drug to precisely repair the pathogenic G>A mutation site of, for example, Hurler syndrome; thereby recovering the normal *in vivo* expression of a protein encoded by RNA, such as IDUA.

## Description

### FIELD OF THE APPLICATION

The present application belongs to the field of gene editing therapeutics, and particularly relates to a method for repairing a disease-causing mutation based on LEAPER (Leveraging Endogenous ADAR for Programmable Editing on RNA) technology for targeted editing of RNA, which includes using LEAPER technology for site-directed base A-to-I editing on RNA to prevent or treat a disease caused by a G>A mutation such as Hurler syndrome.

### BACKGROUND ART

Hurler syndrome, also known as mucopolysaccharidoses IH (MPS IH), is the most severe of the three subtypes of MPSI (IH, IH/S and IS). It is a disabling and lethal inherited metabolic disease caused by the deficiency of α-L-iduronidase (IDUA) in patients with this disease, and is an autosomal recessive (AR) disease. The underlying cause of Hurler syndrome is a mutation in the IDUA gene encoding the IDUA protein at 4p16.3 on human chromosome 4, and to date, there are more than 200 pathogenic mutations for Hurler syndrome, and the most common type among which is a G to A mutation at position 1205 on the cDNAof α-L-iduronidase. This mutation turns the original tryptophan to a stop codon, which in turn causes the final translated protein to lack all amino acids after this site (NM_000203.4(IDUA)-c. 1205G>A (p.Trp402Ter)), thereby losing all enzyme activity of IDUA. This mutation type can account for up to 63% of the total population incidence (Worldwide distribution of common IDUA pathogenic variants, Poletto, Edina (2018). Clinical Genetics. 94. 10.1111/cge.13224). IDUA is responsible for the degradation of glucosaminoglycans (GAGs) within lysosomes. Patients with Hurler syndrome vary widely among individuals, and can be normal at birth. The earliest signs of Hurler syndrome are coarse facial contours at 3-6 months of age, followed by protruding frontal bones, skeletal deformities, growth arrest, and speech disorders, etc., and the patients usually do not live past the age of 10.

There is no cure for Hurler syndrome, and two approved treatments are known as: enzyme replacement therapy (ERT), and hematopoietic stem cell transplantation (HSCT). ERT has shown good results in terms of visceral phenotype, including reduction in liver size, improved respiratory function, and overall improvement in patient mobility. Unfortunately, it does not reach the central nervous system, and therefore does not prevent cognitive impairment. On the other hand, successful HSCT prevents most clinical symptoms, including nervous system symptoms, but the treatment must be performed before the onset of clinical symptoms (preferably before 8 months of age), and this treatment is only suitable for patients with severe disease due to the high mortality rate of it (Combination of enzyme replacement and hematopoietic stem cell transplantation as therapy for Hurler Syndrome.Tolar, J (2008). Bone marrow transplantation. 41. 531-5. 10.1038/sj.bmt.1705934).

The principle of gene therapy for Hurler syndrome currently under investigation is the targeted insertion of a cDNA sequence encoding a normal IDUA protein into the genome of a hepatocyte by using zinc finger nuclease (ZFN) and adeno-associated virus (AAV), but this approach still does not address the symptoms of the brain, the skeletal system, and other systems in Hurler syndrome.

In addition, treatment by DNA editing is one of the relatively promising ideas for therapy development, but the potential for off-targeting is an issue of great concern. In theory, CRISPR (Clustered regularly interspaced short palindromic repeats), a genome editing technology that has been developing rapidly in recent years, could be used to treat Hurler syndrome. Many researchers and biotech companies are also working on bringing the technology to the clinic. For example, in September 2019, the results of clinical trials on using CRISPR technology to edit stem cells and transfuse them back to patients to treat AIDS and leukemia were reported for the first time, making a great contribution to the translation of CRISPR technology in the direction of gene therapy. Although CRISPR technology has great potential applications, it also has a number of drawbacks that make its translation from the research phase to clinical therapeutic applications difficult. One of the problems is the core enzyme used in CRISPR technology: Cas. A CRISPR-based DNA editing technology requires exogenous expression of Cas9 or other nucleases with similar functions, which poses several problems as follows. Firstly, the nucleases that typically require exogenous expression have large molecular weight, making their delivery to the body via viral vectors dramatically less efficient. Secondly, the exogenous expression of the nucleases makes the off-target of the nucleases possible, making their applications potentially oncogenic. Finally, the exogenously expressed nucleases are found from bacteria rather than naturally occurring in humans or mammals, making it possible to induce an immune response in patients, which may on the one hand cause damages to the patients themselves, and on the other hand make the exogenously expressed nucleases to be neutralized and thus lose their proper activity or hinder further intervention and therapy.

In 2017, Feng Zhang's group had reported an RNA editing technique called REPAIR (RNA Editing for Programmable A to I Replacement ) (RNA editing with CRISPR-Cas13, Cox et al., 2017), which also achieves A to I editing of targeted RNAs by exogenous expression of Cas13-ADAR fusion proteins and single guide RNAs (sgRNAs), similar to CRISPR technology, this method still requires expression of exogenous proteins. The problem caused by expression of exogenous proteins cannot be solved.

In 2017, the international application PCT/EP2017/071912 disclosed a more suitable method for *in vivo* editing. This method does not require exogenous proteins, and relies only on the introduction into the cell of a small segment of RNA complementarily paired with the sequence where the target site is located, to recruit ADAR proteins for editing of the target site on RNA. The complementary RNA adopted in this method is short in length (less than 54 nt), but requires complex chemical modifications, and is not efficient for editing.

In January 2019, Thorsten Stafforst's group reported a nucleic acid editing technique called RESTORE (recruiting endogenous ADAR to specific trans for oligonucleotide-mediated RNA editing, Merkle et al., 2019). This technique is also able to get rid of the dependence on exogenous proteins. However, the RESTORE technique requires the presence of IFN-γ to have high editing efficiency, while IFN-γ is a key factor in determining the development and severity of autoimmunity (Interferon-γ and systemic autoimmunity., Pollard et al., 2013), which makes the application of this technique in the medical field greatly diminished.

PCT/CN2018/110105, PCT/CN/2019/082713 and PCT/CN/2019/110782 disclose an RNA editing method called "LEAPER" (Leveraging Endogenous ADAR for Programmable Editing on RNA), a technique that recruits ADAR proteins to edit the target sites on RNA by introducing into the cell a segment of RNA complementarily paired with the sequence at the target site. The RNA complementarily paired with the sequence of the target site used in this technique can stimulate relatively efficient RNA editing at the target site without any modification.

Although there are different methods for gene editing or RNA editing in the prior art, it has been a direction of exploration in the field as to which method can actually achieve the repair of the disease-causing mutation for a specific disease such as Hurler syndrome, and can reach the level of clinical application.

### SUMMARY OF THE APPLICATION

For the Hurler syndrome caused by the G>A mutation in the α-L-iduronidase (IDUA) gene, the present application provides a method for targeted editing of a target RNA containing the mutation in a cell based on LEAPER technology. By the method of the present application, a high efficiency for editing of the G>A mutation in the IDUA gene can be achieved, by correcting the pathogenic G>A mutation the level and activity of IDUA can be restored to some extent, thereby opening a novel avenue for effective treatment of Hurler syndrome.

Particularly, the present application relates to the following:
1. A method for targeted editing of a target RNA in a cell based on the LEAPER technology, wherein the target RNA comprises a transcript sequence of an IDUA gene containing a guanosine (G) to adenosine (A) mutation site (target adenosine), and the method comprises:
   introducing into the cell an arRNA or a construct comprising the arRNA coding sequence, wherein the arRNA comprises a complementary RNA sequence that hybridizes to the target RNA, and wherein the arRNA is capable of recruiting an adenosine deaminase acting on RNA (ADAR) by hybridization to the target RNA so as to deaminate the target adenosine in the target RNA.
2. The method according to item 1, wherein the nucleotide (referred to as targeting nucleotide) in the arRNA opposite to the target adenosine is a cytidine (C), an adenosine (A) or a uridine (U). In some embodiments, the targeting nucleotide opposite to the target adenosine is a C.
3. The method according to item 1 or 2, wherein the target adenosine forms a target triplet together with its 5' most adjacent nucleotide and 3' most adjacent nucleotide, and the target triplet is 5'-UAG-3'.
4. The method according to any one of items 1-3, wherein the 5' most adjacent nucleotide of the targeting nucleotide is a cytidine (C), a guanosine (G) or a uridine (U).
5. The method according to any one of items 1-4, wherein the 3' most adjacent nucleotide of the targeting nucleotide is an adenosine (A).
6. The method according to any one of items 1-5, wherein the targeting nucleotide forms a targeting triplet together with its 5' most adjacent nucleotide and 3' most adjacent nucleotide, and the targeting triplet is 5'-CCA-3'.
7. The method according to any one of items 1-6, wherein the length of the arRNA is greater than 61nt, e.g., the length of the arRNAis 62 to 121nt, 62 to 111nt, 62 to 101nt, 62 to 91nt, 62 to 81nt, or 62 to 76nt. In some embodiments the length of the arRNA is a length of any integer number of nucleotides selected from 62 to 121nt. In some particular embodiments, the length of the arRNAis, for example, 62nt, 63nt, 64t, 65nt, 66nt, 67nt, 68nt, 69nt, 70nt, 71nt, 72nt, 73nt, 74nt, 75nt, 76nt, 77nt, 78nt, 79nt, 80nt, 81nt, 82nt 83nt, 84nt, 85nt, 86nt, 87nt, 88nt, 89nt, 90nt, 92nt, 95nt, 98nt, 100nt, 103nt, or 106nt.
8. The method according to any one of items 1-7, wherein the distance from the targeting nucleotide of the arRNA to the 3' end of the arRNA is less than the distance from the targeting nucleotide to the 5' end of the arRNA. In some embodiments, the distance from the targeting nucleotide of the arRNA to the 3' end of the arRNA equals to the distance from the targeting nucleotide to the 5' end of the arRNA. In some embodiments, the distance from the targeting nucleotide of the arRNA to the 3' end of the arRNAis greater than the distance from the targeting nucleotide to the 5' end of the arRNA.
9. The method according to any one of items 1-8, wherein the distance from the targeting nucleotide of the arRNA to the 3' end of the arRNAis greater than 8nt, e.g., 9 to 60nt, preferably 9-45nt or 9-35nt; in some embodiments, in order to avoid an extra-long poly G structure, the distance from the targeting nucleotide of the arRNA to the 3' end of the arRNAis selected from: 9 to 25nt, 9 to 26nt, or 9 to 27nt; preferably, in some embodiments, the distance from the targeting nucleotide of the arRNA to the 3' end of the arRNA is selected from: 9 to 20nt, 9 to 21nt, or 9 to 22nt; in some particular embodiments, the distance from the targeting nucleotide of the arRNA to the 3' end of the arRNAis an integer number of nucleotides selected from 9 to 25nt, e.g., 10nt, 11nt, 12nt, 13nt, 14nt, 15nt, 16nt, 17nt, 18nt, 19nt, 23nt, or 24nt.
10. The method according to any one of items 1-9, wherein the distance from the targeting nucleotide of the arRNA to the 5' end of the arRNAis greater than 25nt, e.g., 26 to 60nt, 35 to 60nt, or 45 to 60nt; in some preferred embodiments, the distance from the targeting nucleotide of the arRNA to the 5' end of the arRNA is selected from the group consisting of: 45nt, 45nt, 46nt, 47nt, 48nt, 49nt, 50nt, 51nt, 52nt, 53nt, 54nt, 55nt, 56nt, 57nt, 58nt, or 59nt.
11. The method according to any one of items 1-7, wherein the distance from the targeting nucleotide of the arRNA to the 5' end of the arRNA is less than the distance from the targeting nucleotide of the arRNA to the 3' end of the arRNA.
12. The method according to item 11, wherein tthe distance from the targeting nucleotide of the arRNA to the 5' end of the arRNA is greater than 35nt, e.g., 36 to 60nt, or 36 to 55nt; preferably, the distance from the targeting nucleotide of the arRNA to the 5' end of the arRNA is selected from the group consisting of: 54nt, 53nt, 52nt, 51nt, 50nt, 49nt, 48nt, 47nt, 46nt, 45nt, 44nt, 43nt, 42nt, 41nt, 40nt, 39nt, 38nt or 36nt.
13. The method according to any one of items 1-12, wherein the arRNA is an arRNA having a length charactorastic selected from the group consisting of:
   55nt-c-35nt, 55nt-c-25nt, 55nt-c-24nt, 55nt-c-21nt, 55nt-c-20nt, 55nt-c-19nt, 55nt-c-18nt, 55nt-c-17nt, 55nt-c-16nt, 55nt-c-15nt, 55nt-c 14nt, 55nt-c-13nt, 55nt-c-12nt, 55nt-c-11nt, 55nt-c-10nt, 55nt-c-9nt, 50nt-c-20nt, 50nt-c-15nt, 45nt-c-55nt, or 45nt-c-20nt.
14. The method according to any one of items 1-13, wherein the arRNA is an arRNA having a length charactorastic selected from the group consisting of: 55nt-c-20nt, 55nt-c-15nt, or 55nt-c-14nt.
15. The method according to any one of items 1-14, wherein the arRNA comprises one or more chemical modifications.
16. The method according to item 15, wherein the chemical modification is one or more selected from the group consisting of:
   2'-O-methylation modification, phosphorothioation modification, deoxyribonucleotide substitution modification, LNA modification, and 2'-O-(2-methoxyethyl) modification.
17. The method according to item 15 or 16, wherein the chemical modification is one or more selected from the group consisting of:
   1) 2'-O-methylation modifications in the first 2, 3, 4 or 5 nucleotides;
   2) 2'-O-methylation modifications in the last 2, 3, 4, or 5 nucleotides;
   3) 2'-O-methylation modifications in all the cytidines except that in the targeting triplet;
   4) 2'-O-(2-methoxyethyl) modifications in the first 2, 3 or 4 nucleotides;
   5) 2'-O-(2-methoxyethyl) modifications in the last 2, 3 or 4 nucleotides;
   6) deoxyribonucleotide substitution modifications of the first 2, 3 or 4 nucleotides;
   7) deoxyribonucleotide substitution modifications of the last 2, 3 or 4 nucleotides;
   8) phosphorothioation modifications in the first 1, 2, 3, 4 or 5 internucleotide linkages;
   9) phosphorothioation modifications in the last 1, 2, 3, 4 or 5 internucleotide linkages;
   10) phosphorothioation modifications in all internucleotide linkages except that one or more of the 5' or 3' most adjacent internucleotide linkages of each of the nucleotides in the targeting triplet are not modified by phosphorothioation; and
   11) phosphorothioation modifications in internucleotide linkages at an interval of 1, 2, 3, or 4 nucleotides except that one or more of the 5' or 3' most adjacent internucleotide linkages of each of the nucleotides in the targeting triplet are not modified by phosphorothioation.
18. The method according to item 17, wherein the chemical modification is one or more selected from the group consisting of:
   1) 2'-O-methylation modifications in the first 2, 3, 4 or 5 nucleotides and the last 2, 3, 4, or 5 nucleotides;
   2) 2'-O-methylation modifications in all the cytidines except that in the targeting triplet;
   3) 2'-O-(2-methoxyethyl) modifications in the first 2, 3 or 4 nucleotides and the last 2, 3 or 4 nucleotides;
   4) deoxyribonucleotide substitution modifications in the first 2, 3 or 4 nucleotides and the last 2, 3 or 4 nucleotides;
   5) phosphorothioation modifications in the first 1, 2, 3, 4 or 5 internucleotide linkages and the last 1, 2, 3, 4 or 5 internucleotide linkages;
   6) phosphorothioation modifications in all internucleotide linkages, or at least every other internucleotide linkage, except that one or more of the 5' or 3' most adjacent internucleotide linkages of the nucleotides in the targeting triplet are not modified by phosphorothioation.
19. The method according to any one of items 15-18, wherein the arRNA comprises any chemical modification selected from the group consisting of:
   CMC: the first 3 and the last 3 nucleotides in the arRNA sequence are respectively modified by 2'-O-methylation, and the first 3 and the last 3 internucleotide linkages in the arRNA sequence are respectively modified by phosphorothioation;
   CM1: the first 3 and the last 3 nucleotides in the arRNA sequence are respectively modified by 2'-O-methylation, the first 3 and the last 3 internucleotide linkages in the arRNA sequence are respectively modified by phosphorothioation, and all the uridines of the sequence are modified by 2'-O-methylation;
   CM2: the first 3 and the last 3 nucleotides in the arRNA sequence are respectively modified by 2'-O-methylation, and the first 3 and last 3 internucleotide linkages in the arRNA sequence are respectively modified by phosphorothioation; and the 3' most adjacent nucleotide of the targeting nucleotide is modified by 2'-O-methylation;
   CM3: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 5' most adjacent nucleotide of the targeting nucleotide is modified by 2'-O-methylation;
   CM4: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 3' most adjacent internucleotide linkage of each of the three bases in the targeting triplet is respectively modified by phosphorothioation;
   CM6: the first 5 and the last 5 nucleotides in the arRNA sequence are respectively modified by 2'-O-methylation, and the first 5 and the last 5 internucleotide linkages in the arRNA sequence are respectively modified by phosphorothioation;
   CM148: the first 4 and the last 4 nucleotides in the arRNA sequence are respectively modified by 2'-O-(2-methoxyethyl), the first 3 and the last 4 internucleotide linkages in the arRNA sequence are respectively modified by phosphorothioation, and all the uridines of the sequence are modified by 2'-O-Me;
   CM149: the first 3 and the last 3 nucleotides in the arRNA sequence are respectively modified by 2'-O-(2-methoxyethyl), the first 2 and the last 3 internucleotide linkages in the arRNA sequence are respectively modified by phosphorothioation, and all the uridines of the sequence are modified by 2'-O-Me;
   CM150: the first 2 and the last 2 nucleotides in the arRNA sequence are respectively modified by 2'-O-(2-methoxyethyl), the first 1 and the last 2 internucleotide linkages in the arRNA sequence are respectively modified by phosphorothioation, and all the uridines of the sequence are modified by 2'-O-Me;
   CM151: the first 4 and the last 4 nucleotides in the arRNA sequence are respectively modified by LNA, the first 3 and the last 4 internucleotide linkages in the arRNA sequence are respectively modified by phosphorothioation, and all the uridines of the sequence are modified by 2'-O-Me;
   CM152: the first 3 and the last 3 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
   CM153: the first 2 and the last 2 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
   CM94: the first 3 and the last 3 nucleotides in the arRNA sequence, all the uridines and all the cytidines except that in the targeting triplet in the arRNA sequence are modified by 2'-O-methylation; the first 4 and the last 3 internucleotide linkages in the arRNA sequence are all modified by phosphorothioation; and from the 4th internucleotide linkage from the 5' end to the 3rd internucleotide linkage from the 3' end, every other internucleotide linkage is modified by phosphorothioation, except that the 5' most adjacent internucleotide linkage of each nucleotide in the targeting triple is not modified by phosphorothioation;
   CM119: the first 3 and the last 3 nucleotides in the arRNA sequence, all the uridines and all the cytidines in the arRNA sequence except that in the targeting triplet, are modified by 2'-O-methylation; the first 4 and the last 3 internucleotide linkages in the arRNA sequence are all modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and the targeting nucleotide is substituted by a deoxyribonucleotide;
   CM120: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages in the arRNA sequence are modified by phosphorothioation; from the 4th internucleotide linkage from the 5' end to the 5' most adjacent internucleotide linkage of the targeting nucleotide, and from the 3rd internucleotide linkage from the 3' end to the 3' most adjacent internucleotide linkage of the targeting nucleotide, every other internucleotide linkage is modified by phosphorothioation; and the targeting nucleotide is substituted by a deoxyribonucleotide;
   CM123: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages are modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide;
   CM125: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages and the 3' most adjacent nucleotide linkages of the targeting nucleotide are modified by phosphorothioation; from the 4th internucleotide linkage from the 5' end to the 5' most adjacent nucleotide of the targeting nucleotide's 5' most adjacent nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide.
20. The method according to any one of items 1-19, wherein the guanosine (G) to adenosine (A) mutation site targeted by the arRNA is a mutation site in NM_000203.4(IDUA)-c.1205G>A (p.Trp402Ter), or a corresponding mutation site thereof, in the genome of a patient with Hurler syndrome.
21. The method according to any one of items 1-20, wherein the arRNA comprises a sequence selected from the group consisting of: 
1)Gm*Am*Cm-G*Cm-Cm*Cm-A*Cm-Cm*G-Um*G-Um*G-G*Um-Um*G-Cm*Um-G*Um-
Cm*Um-Cm*Cm-A*G-G*A-Cm*G-G*Um-Cm*G-G*Um-Cm*Cm-Cm*G-G*Cm-Cm*Um-G*Cm
-Cm*Um-G*Cm-G*Cm-G*A-Cm*A-Cm*A-Cm*Um-Um*Cm-G*G-Cm-C-C-A*G-A*G-Cm*Um-
G*Cm-Um*Cm-Cm*Um*Cm*Cm*Am (SEQ ID NO: 31);
 
2)Gm*Am*Cm-G*Cm-Cm*Cm-A*-Cm-Cm*G-Um*G-Um*G-G*Um-Um*G-Cm*Um-G*Um
-Cm*CM-A*G-G*A-Cm*G-G-G*Um-Cm*G*Cm*G-G*Um-Cm*Cm-Cm*G-G*Cm-Cm*Um-G*C
m*Um-G*G*-A-Cm*A-Cm*A-Cm*Um-Um*Cm-G*G-Cm*Cd-Cd*Ad*-G-A*G-Cm*Um-G*Cm-
Um*Cm-Cm*Um*Cm*Am (SEQ ID NO: 31);
 
3)Gm*Am*Cm-G*Cm-Cm*Cm-A*Cm-Cm*G-Um*G-Um*G-G*Um-Um*G-G*Um-Um*G-C
m*Um-G*Um-Cm*Cm-A*G-G*A-Cm*-G-G*Um-Cm*-Cm*-G*Um-Cm*G-G*Cm-Cm*Um-G*G-
Cm*Um-G*-Cm*Um-G*-Cm-G*A-Cm*A-Cm*A-Cm*Um-Um*Cm-G*G-Cm-Cd-Cd*Ad*G-A*G-
Cm*Um-G*Cm-Um*Cm-Cm*Um*Cm*Am (SEQ ID NO: 31);
 4)Gmoe*Amoe*Cmoe*Gmoe-C-C-C-A-C-C-C-G-Um-G-Um-G-G-Um-Um-G-C-Um-G-Um-C 
-C-A-G-G-G-Um-G-Um-C-C-A-G-G-G-A-G-A-C-G-G-G-Um-C-C-G-Gm-G-C-C-C-A-G-A-G-C-U
m-G-C-Um-C*Cmoe*Umoe*Cmoe*Amoe (SEQ ID NO: 31); or
 
5)G+*A+*C+*G+C-C-C-A-C-C-C-G-Um-G-Um-G-G-Um-Um-G-C-Um-G-Um-C-C-A-G-G-
A-G-G-A-A-G-G-A-C-C-G-G-Um-G A-G-C-Um-G-C-Um-C*C+*U+*C+*A+ (SEQ ID NO: 31).
22. The method according to any one of items 1-21, wherein the arRNA is introduced into the cells by a method selected from the group consisting of: electroporation, liposome transfection, exosome delivery, or lipid-nanoparticle delivery (LNP).
23. The method according to any one of items 1-22, wherein the amount of arRNA in a single introduction into the cells is ≥5nM, preferably ≥10nM, further preferably ≥20nM; in some particular embodiments, the amount of arRNA in a single introduction into the cells is 5-160nM, e.g., 10-160nM, 20-160nM, 40-160nM, 20-80nM, 40-80nM, or 20-40nM; in some particular embodiments, the amount of arRNA in a single introduction into the cells is 15nM, 17nM, 19nM, 21nM, 22nM, 23nM, 24nM, 25nM, 26nM, 27nM, 28nM, 29nM, 30nM, 31nM, 32nM, 33nM, 34nM, 35nM, 36nM, 37nM, 38nM, 39nM, 41nM, 43nM, 45nM, 55nM, 65nM, 75nM, 85nM, 95nM, 105nM, 115nM, 125nM, 135nM, 145nM, or 155nM.
24. The method according to any one of items 1-23, wherein several introductions are taken for introducing the arRNA into the cells, and the interval between the adjacent two introductions is ≤ 21 days, e.g., >_ 17 days, >_ 14 days, >_ 12 days, >_ 11 days, or ≥ 10 days; in some embodiments, the interval between the adjacent two introductions is 9-20 days, 9-16 days, 9-14 days, 12-19 days, 12-15 days, or 12- 13 days; in some embodiments, the interval between the adjacent two introductions is 7 days, 8 days, 11 days, 15 days, 18 days, 19 days, or 20 days.
25. A method for preventing or treating Hurler syndrome, comprising correcting a pathogenic G>A mutation of Hurler syndrome by using the method according to any one of items 1-24.
26. An engineered arRNA which is used for targeted editing of a target RNA in a cell based on LEAPER technology, wherein the target RNA comprises a transcript sequence of an IDUA gene containing a guanosine (G) to adenosine (A) mutation site (target adenosine), the arRNA comprises a complementary RNA sequence that hybridizes to the target RNA, and the arRNA is capable of recruiting an adenosine deaminase acting on RNA (ADAR) by hybridization to the target RNA so as to deaminate the target adenosine in the target RNA, the guanosine (G) to adenosine (A) mutation site targeted by the arRNA is NM_000203.4(IDUA)-c.1205G>A (p.Trp402Ter) mutation site, or a corresponding mutation site thereof, in the genome of a patient with Hurler syndrome.
27. The arRNA of item 26, wherein the nucleotide opposite the target adenosine (targeting nucleotide) is a cytidine (C), an adenosine (A), or a uridine (U).
28. The arRNA of item 27, wherein the targeting adenosine forms a targeting triplet together with its 5' most adjacent nucleotide and 3' most adjacent nucleotide, and the targeting triplet is 5'-CCA-3'.
29. The arRNA of any one of items 26-28, wherein the arRNA is an arRNA having a length charactorastic selected from the group consisting of:
   55nt-c-35nt, 55nt-c-25nt, 55nt-c-24nt, 55nt-c-21nt, 55nt-c-20nt, 55nt-c-19nt, 55nt-c-18nt, 55nt-c-17nt, 55nt-c-16nt, 55nt-c-15nt, 55nt-c-14nt, 55nt-c-13nt, 55nt-c-12nt, 55nt-c-11nt, 55nt-c-10nt, 55nt-c-9nt, 50nt-c-20nt, 50nt-c-15nt, 45nt-c-55nt, or 45nt-20nt,
   wherein cytidine is the targeting nucleotide in the arRNA opposite to the target adenosine, and the orientation of the arRNA is 5' to 3'.
30. The arRNA according to any one of items 26-29, wherein the arRNA comprises one or more chemical modifications.
31. The arRNA according to any one of items 26-30, wherein the chemical modification is one or more selected from the group consisting of:
   2'-O-methylation modification, phosphorothioation modification, deoxyribonucleotide substitution modification, LNA modification, and 2'-O-(2-methoxyethyl) modification.
32. The arRNA according to any one of items 26-31, wherein the chemical modification is one or more selected from the group consisting of:
   1) 2'-O-methylation modifications in the first 2, 3, 4 or 5 nucleotides;
   2) 2'-O-methylation modifications in the last 2, 3, 4, or 5 nucleotides;
   3) 2'-O-methylation modifications in all the cytidines except that in the targeting triplet;
   4) 2'-O-(2-methoxyethyl) modifications in the first 2, 3 or 4 nucleotides;
   5) 2'-O-(2-methoxyethyl) modifications in the last 2, 3 or 4 nucleotides;
   6) deoxyribonucleotide substitution modifications of the first 2, 3 or 4 nucleotides;
   7) deoxyribonucleotide substitution modifications of the last 2, 3 or 4 nucleotides;
   8) phosphorothioation modifications in the first 1, 2, 3, 4 or 5 internucleotide linkages;
   9) phosphorothioation modifications in the last 1, 2, 3, 4 or 5 internucleotide linkages;
   10) phosphorothioation modifications in all internucleotide linkages except that one or more of the 5' or 3' most adjacent internucleotide linkages of each of the nucleotides in the targeting triplet are not modified by phosphorothioation; and
   11) phosphorothioation modifications in internucleotide linkages at an interval of 1, 2, 3, or 4 nucleotides except that one or more of the 5' or 3' most adjacent internucleotide linkages of each of the nucleotides in the targeting triplet are not modified by phosphorothioation.
33. The arRNA according to any one of items 26-32, wherein the arRNA comprises any chemical modification selected from the group consisting of:
   CMC: the first 3 and the last 3 nucleotides in the arRNA sequence are respectively modified by 2'-O-methylation, and the first 3 and the last 3 internucleotide linkages in the arRNA sequence are respectively modified by phosphorothioation;
   CM1: the first 3 and the last 3 nucleotides in the arRNA sequence are respectively modified by 2'-O-methylation, the first 3 and the last 3 internucleotide linkages in the arRNA sequence are respectively modified by phosphorothioation, and all the uridines of the sequence are modified by 2'-O-methylation;
   CM2: the first 3 and the last 3 nucleotides in the arRNA sequence are respectively modified by 2'-O-methylation, and the first 3 and last 3 internucleotide linkages in the arRNA sequence are respectively modified by phosphorothioation; and the 3' most adjacent nucleotide of the targeting nucleotide is modified by 2'-O-methylation;
   CM3: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 5'-most adjacent nucleotide of the targeting nucleotide is modified by 2'-O-methylation;
   CM4: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 3' most adjacent internucleotide linkage of each of the three bases in the targeting triplet is respectively modified by phosphorothioation;
   CM6: the first 5 and the last 5 nucleotides in the arRNA sequence are respectively modified by 2'-O-methylation, and the first 5 and the last 5 internucleotide linkages in the arRNA sequence are respectively modified by phosphorothioation;
   CM148: the first 4 and the last 4 nucleotides in the arRNA sequence are respectively modified by 2'-O-(2-methoxyethyl), the first 3 and the last 4 internucleotide linkages in the arRNA sequence are respectively modified by phosphorothioation, and all the uridines of the sequence are modified by 2'-O-Me;
   CM149: the first 3 and the last 3 nucleotides in the arRNA sequence are respectively modified by 2'-O-(2-methoxyethyl), the first 2 and the last 3 internucleotide linkages in the arRNA sequence are respectively modified by phosphorothioation, and all the uridines of the sequence are modified by 2'-O-Me;
   CM150: the first 2 and the last 2 nucleotides in the arRNA sequence are respectively modified by 2'-O-(2-methoxyethyl), the first 1 and the last 2 internucleotide linkages in the arRNA sequence are respectively modified by phosphorothioation, and all the uridines of the sequence are modified by 2'-O-Me;
   CM151: the first 4 and the last 4 nucleotides in the arRNA sequence are respectively modified by LNA, the first 3 and the last 4 internucleotide linkages in the arRNA sequence are respectively modified by phosphorothioation, and all the uridines of the sequence are modified by 2'-O-Me;
   CM152: the first 3 and the last 3 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
   CM153: the first 2 and the last 2 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
   CM94: the first 3 and the last 3 nucleotides in the arRNA sequence, all the uridines and all the cytidines except that in the targeting triplet in the arRNA sequence are modified by 2'-O-methylation; the first 4 and the last 3 internucleotide linkages in the arRNA sequence are all modified by phosphorothioation; and from the 4th internucleotide linkage from the 5' end to the 3rd internucleotide linkage from the 3' end, every other internucleotide linkage is modified by phosphorothioation, except that the 5' most adjacent internucleotide linkage of each nucleotide in the targeting triple is not modified by phosphorothioation;
   CM119: the first 3 and the last 3 nucleotides in the arRNA sequence, all the uridines and all the cytidines in the arRNA sequence except that in the targeting triplet, are modified by 2'-O-methylation; the first 4 and the last 3 internucleotide linkages in the arRNA sequence are all modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and the targeting nucleotide is substituted by a deoxyribonucleotide;
   CM120: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages in the arRNA sequence are modified by phosphorothioation; from the 4th internucleotide linkage from the 5' end to the 5' most adjacent internucleotide linkage of the targeting nucleotide, and from the 3rd internucleotide linkage from the 3' end to the 3' most adjacent internucleotide linkage of the targeting nucleotide, every other internucleotide linkage is modified by phosphorothioation; and the targeting nucleotide is substituted by a deoxyribonucleotide;
   CM123: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages are modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide;
   CM125: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages and the 3' most adjacent nucleotide linkages of the targeting nucleotide are modified by phosphorothioation; from the 4th internucleotide linkage from the 5' end to the 5' most adjacent nucleotide of the targeting nucleotide's 5' most adjacent nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide.
34. The arRNA according to any one of itms 26-33, wherein the arRNA comprises any sequence selected from the group consisting of: 
1)Gm*Am*Cm*G*Cm-Cm*Cm-A*Cm-Cm*G-Um*G-Um*G-G*Um-Um*G-Cm*Um-G*Um-
Cm*Cm-A*G-G*A-Cm*G-G*Um-Cm*Cm-Cm*G-G*Cm-Cm*Um-G*Cm-G*A-Cm*A-Cm*Um-U
m*Cm-G*G-Cm-C-C-A*G-A*G-Cm*Um-G*Cm-Um*Cm-Cm*Um*Cm*Am (SEQ ID NO: 31);
 
2)Gm*Am*Cm*G*Cm-Cm*Cm-A*-Cm-Cm*G-Um*G-Um*G-G*Um-Um*G-Cm*Um-G*Um
-Cm*Cm-A*G-G*A-Cm*G-G*Um-Cm*Cm-Cm*G-G*Cm-Cm*Um-G*Cm-G*-A-Cm*A-Cm*Um-
Um*Cm-G*G-Cm*Cd-Cd*Ad*-G-A*G-Cm*Um-G*Cm-Um*Cm-Cm*Um*Cm*Am (SEQ ID NO:
31);
 
3)Gm*Am*Cm*G*Cm-Cm*Cm-A*Cm-Cm*G-Um*G-Um*G-G*Um-Um*G-Cm*Um-G*Um-
Cm*Cm-A*G-G*A-Cm*-G-G*Um-Cm*Cm-Cm*G-G*Cm-Cm*Um-G*-Cm-G*A-Cm*A-Cm*Um-
Um*Cm-G*G-Cm-Cd-Cd*Ad*G-A*G-Cm*Um-G*Cm-Um*Cm-Cm*Um*Cm*Am (SEQ ID NO:
31);
 
4)Gmoe*Amoe*Cmoe*Gmoe-C-C-C-A-C-C-G-Um-G-Um-G-G-Um-Um-G-C-Um-G-Um-C-C
-A-G-G-A-C-G-G-Um-C-C-C-G-G-C-C-Um-GC-G-A-C-A-C-Um-Um-C-G-G-C-C-A-G-A-G-C-
Um-G-C-Um-C*Cmoe*Umoe*Cmoe*Amoe (SEQ ID NO: 31); or
 
5)G+*A+*C+*G+C-C-C-A-C-C-G-Um-G-Um-G-G-Um-Um-G-C-Um-G-Um-C-C-A-G-G-A-
C-G-G-Um-C-C-C-G-G-C-C-Um-GC-G-A-C-A-C-Um-Um-C-G-G-C-C-C-A-G-A-G-C-Um-G-C-U
m-C*C+*U+*C+*A+ (SEQ ID NO: 31).
35. A construct comprising the coding sequence of the arRNA according to any one of items 26-29.
36. A composition, liposome, exosome, lipid-nanoparticle, cell, library, product or kit comprising the arRNA according to any one of items 26-34 or the construct according to item 35.
37. Use of the arRNA according to any one of items 26-34, the construct according to item 35, and the composition, liposome, exosome, lipid-nanoparticle, cell, library, or kit according to item 36 in the preparation of a drug, product, or kit for preventing or treating Hurler syndrome.

In some embodiments, the arRNA comprises a sequence complementary to an IDUA encoding RNA sequence, and comprises one or more mismatches, wobble pairings and/or unilateral bulges.

In the present application, the arRNA can be expressed in the format of Xnt-c-Ynt (in the order from the 5' to the 3' end), wherein X indicates the number of nucleotides from the targeting nucleotide (not included) to the last nucleotide (included) at the 5' end of the arRNA, and Y indicates the number of nucleotides from the targeting nucleotide (not included) to the last nucleotide (included) at the 3' end of the arRNA. For example, when the arRNA is 55nt-c-35nt, it means that distance from the targeting nucleotide to the 5' end of the arRNA is 55 nucleotides, and the distance from the targeting nucleotide to the 3' end of the arRNA is 35 nucleotides, and the length of the entire arRNA is 55nt + 35nt + 1nt = 91nt.

In some embodiments, the arRNA does not comprise a region capable of forming an intramolecular stem-loop structure for recruiting the ADAR enzyme. In some embodiments, the method for targeted editing of a target RNA in a cell based on the LEAPER technology does not comprise introducing an exogenous ADAR enzyme protein into the cell. In some embodiments, the method for targeted editing of a target RNA in a cell based on the LEAPER technology comprises introducing an exogenous ADAR enzyme protein into the cell.

By using the arRNA described herein, the validation at the cellular level reveals that the recovery rate of the c. 1205G>A mutant site (A>G editing efficiency) on the RNA encoding the IDUA gene is at least about 30%, e.g., at least about 30%, 35%, 40%, 45%, 50%, 55%, 60%, 65%, 70%, 75%, 80%, 85%, 90% or higher.

The method described herein for treating or preventing Hurler syndrome, wherein the arRNA does not induce a strong immune response in the body.

In summary, the present application provides an arRNA for treating Hurler syndrome, and the construct, composition, cell, library or kit comprising the arRNA, and the method for preventing and treating Hurler syndrome by using the arRNA, construct, composition, cell, library or kit. The technical solutions of the present application have at least the following advantages:
1. Not dependent on the expression of exogenous proteins. Thus, there is no cell damage due to introduction of exogenous proteins; no off-target effects due to overexpression of exogenous proteins; no immune response in the body caused by exogenous proteins; no gene editing failure due to neutralization of effective exogenous proteins caused by preexisting antibodies in the body; and no limitation of the choice of delivery vectors due to the excessive molecular weight of the proteins to be introduced.
2. The present application provides an RNA editing method which, unlike DNA editing, is reversible and adjustable, and does not damage genomic DNA to cause irreversible damage, and is therefore safer.
3. The technical solution provided in this application is more efficient in editing compared to other treatment solutions applicable *in vivo* gene editing.

### BRIEF DESCRIPTION OF THE DRAWINGS

Fig. 1 shows the detected IDUA genotype (c1205 G>A) in GM06214 cells.
Figs. 2A-2B show the design of arRNA for IDUA pre-mRNA and mRNA. Functions of the Cells edited by these arRNAs (arRNAs are introduced into the cells by electroporation) were tested; and the A>G editing efficiency of the editing site was tested.
Figs. 3A-3B show the design of the IDUA-reporter cell line (3A), and the editing efficiency of arRNAs of different lengths (symmetrically truncated) on the 293T-IDUA-reporter was detected (arRNAs are introduced into the cells by electroporation) (3B).
Figs. 4A-4B show the enzyme activity and editing efficiency at different time points after electroporation of arRNAs of different lengths (symmetrically truncated) in GM06214 cells.
Fig. 5 shows the detected IDUA enzyme activity and editing efficiency after transfection of arRNAs (symmetrically truncated, 3' truncated and 5' truncated) into GM06214 cells by lipofectamineRNAiMAX.
Figs. 6A-6B show the preferred arRNAs from 55nt-c-25nt to 55nt-c-5nt (base by base reduction at 3' end), which were transfected into GM06214 cells by lipofectamineRNAiMAX, and the preferred 3' end length range was selected by enzyme activity detection. Fig. 6A shows the detection results of the arRNAs from 55nt-c-25nt to 55nt-c-10nt. Fig. 6B shows another synthetic batch of arRNAs from 55nt-c-16nt to 55nt-c-5nt used herein.
Fig. 7 shows transfection of arRNAs with the same length at the 3' end (fixed at two preferred lengths) and varying lengths at the 5' end (gradually reducing bases from 55nt) into GM06214 cells by lipofectamineRNAiMAX, and the preferred 5' end length range was screened for by enzyme activity detection. The figure also shows that the editing efficiency of arRNA is significantly reduced when it is less than 60nt.
Fig. 8 shows limited shifts of the editing sites of the arRNAs with 3 preferred lengths were performed to produce 3 groups of arRNAs. arRNAs in each group are of the same length, and have different editing sites. These arRNAs were transfected into GM06214 cells respectively by using lipofectamineRNAiMAX transfection, and the best combination of length and editing sites was screened according by enzyme activity detection.
Figs. 9A-9C show comparison of the effects of CM1 and CM0 modifications on arRNA editing efficiency at three arRNA lengths (Fig. 9A), and transfection of arRNAs of 71nt and 76nt containing different chemical modifications into GM06214 cells with lipofectamineRNAiMAX, and the preferred chemical modifications were screened by enzyme activity detection (Fig. 9B) and detection of the A>G mutation rate on the target site (Fig. 9C).
Figs. 10A-10B show the concentration gradient test of CM1 modified 55nt-c-16nt, 55nt-c-14nt, and 55nt-c-11nt. The effects of the arRNAs were observed in terms of enzyme activity (Fig. 10A) and mutation rate on the target site (Fig. 10B), respectively.
Figs. 11A-11B show transfection of the arRNAs into GM06214 cells by lipofectamineRNAiMAX, and the effect of CM1 modification was further verified by enzyme activity detection.
Fig. 12 shows the editing efficiency of A>G mutation in wild-type IDUA gene in human liver primary cells.
Fig. 13 shows comparison of the editing efficiency (represented by IDUA enzyme activity) of the transcript of c.1239 G>A (p.Trp402Ter) mutation site in GM06214 cells between the method according to the present application and a preferred method in the prior art.
Figs. 14A-14B show the effect of different types and amounts of terminal modifications on the results of G>A gene editing of arRNA, which are presented as the fluorescent expressions of cells containing the reporter system on day 2 (Fig. 14A) and day 7 (Fig. 14B).
Figs. 15A-15C show the effect of combinations of different amounts and different base sites of phosphorothioation modification, 2'-O-Me modification and deoxyribonucleotide substitution modification on the results of G>A gene editing of an arRNA, which are presented as the fluorescent expressions in cells containing the reporter system on day 3 (Fig. 15A), day 5 (Fig. 15B), and day 7 (Fig. 15C).

### DETAIL DESCRIPTION OF THE APPLICATION

### Definitions

The present application will be described herein by giving examples and with reference to the drawings, but the present application is not limited thereto, but is defined only by the claims. Any appended drawing reference markings in the claims should not be construed as limiting the scope. When the term "comprise/include" or "comprising/including" is used in the specification and claims, they do not exclude other components, elements or steps. When a numerical range of nucleotides is used herein, the values of the two endpoints of the range and all integers between the two endpoints are included. For example, for the range of 60-200 nucleotides, any integer number of nucleotides between 60 and 200 nucleotides is covered in addition to the numbers of 60 nucleotides and 260 nucleotides.

The following terms or definitions are provided only to aid in the understanding of the present application. Unless specifically defined herein, all terms used herein have the same meaning as those used by those skilled in the art of the application. Practitioners may rely on books e.g., Molecular Cloning: a Laboratory Manual (Fourth Edition), (March 2017, published by Science Press, LLC, authors J. Sambrook, M.R. Green, and translator, Fuchu He) to understand definitions and terms in the field. The definitions provided herein should not be construed as having a scope less than that understood by those of ordinary skill in the art.

As used herein, the term "target site" refers to the nucleotide site in the RNA sequence to be edited.

The term "dRNA" (Deaminase Recruiting RNA) refers to an engineered RNA capable of recruiting a deaminase e.g., adenosine deaminase acting on RNA (ADAR) to deaminate a target adenosine. It is also referred to as arRNA" (Adenosine Deaminase Recruiting RNA) due to its function of recruiting an adenosine deaminase. In the present application dRNA and arRNA are used interchangeably to denote the same meaning. For example, in the context of the present application, arRNA binds to a segment of RNA in a cell through base complementary pairing and recruits a deaminase, e.g., ADAR, to deaminate a target nucleotide, e.g., adenosine (A), located at the target site of that RNA sequence into, e.g., inosine, thereby enabling editing. Particularly, when the target nucleotide is an adenosine, the target nucleotide is also referred to as "target adenosine" or "target A". The RNA comprising the target nucleotide is referred to as "target RNA" or "targeted RNA" and the sequence comprising the target nucleotide in the target RNA is referred to as the "target sequence" or "targeted sequence".

In the present application, when the arRNA used binds and hybridizes to the target RNA, one nucleotide in the arRNA sequence is directly opposite the target adenosine to be edited in the target RNA. This nucleotide in the arRNA sequence can be a cytidine (C), an adenosine (A) or a uridine (U). The cytidine (C), adenosine (A) and uridine (U) directly opposite the target adenosine are collectively referred to as the "targeting nucleotide" or respectively referred to as the "targeting cytidine (C)", "targeting adenosine (A)" and "targeting uridine (U)".

In this application, all nucleic acid sequences are written in the order from the 5' to the 3' end. Therefore, when "first" and "last" are used to describe a sequence in this document, they denote the "5' end" and the "3' end" of the the sequence, respectively. Particularly, the nucleotide nearest to any nucleotide in the direction of its 5' end is called the "5' most adjacent nucleotide" or "5' most adjacent nucleoside" of the nucleotide; the nucleotide nearest to any nucleotide in the direction of its 3' end is called the "3' most adjacent nucleotide" or "3' most adjacent nucleoside" of the nucleotide. The linkage between any of the nucleotides and its 5' most adjacent nucleotide is referred to as a "5' most adjacent internucleotide linkage"; the linkage between any of the nucleotides and its 3' most adjacent nucleotide is referred to as a "3' most adjacent internucleotide linkage". For example, the most adjacent nucleotides in the 5' and 3' end directions of a target nucleotide in a target RNA are referred to as "5' most adjacent nucleotide of the target nucleotide" and "3' most adjacent nucleotide of the target nucleotide" respectively; and a nucleotide triplet formed by linking the target nucleotide and its 5' most adjacent nucleotide and 3' most adjacent nucleotide is called "target triplet". Similarly, the most adjacent nucleotides in the direction of the 5' and 3' ends of the targeting nucleotide are called "5' most adjacent nucleotide of the targeting nucleotide" and "3' most adjacent nucleotide of the targeting nucleotide" respectively; and a nucleotide triplet formed by linking the targeting nucleotide and its 5' most adjacent nucleotide and 3' most adjacent nucleotide is called "targeting triplet".

The terms "polynucleotide", "nucleotide sequence" and "nucleic acid" are used interchangeably. They refer to polymeric forms of nucleotides of any length, i.e., the polymeric forms of deoxyribonucleotides (DNA) or ribonucleotides (RNA) or the analogs thereof. The deoxyribonucleotide or ribonucleotide may be referred to as "nucleotide" or "nucleoside". In this application, unless otherwise indicated, "nucleotide" and "nucleoside" are used interchangeably. Nucleotides are linked to each other by phosphodiester bonds. A plurality of nucleotides are linked by phosphodiester bonds to form a polynucleotide or nucleic acid. Phosphodiester bonds between nucleotides can be phosphorthioated, replacing a double bonded oxygen in the phosphodiester bond with a double bonded sulfur to form a "phosphorothioate bond", and this process is known as "phosphorothioation modification".

In this application, "2'-O-Me" or "2'-OMe", i.e. 2-O'-methyl, refers to the group formed by replacing the hydrogen atom on the second hydroxyl group on the ribose of a nucleotide with a methyl group. And this process of the methyl substitution is referred to as "2'-O-methylation", "2'-O-methylation modification", "2'-OMe modification" or "2'-O-Me modification". As used herein, the term "LNA" or "locked nucleic acid" refers to a specific bicyclic nucleotide derivative, in which the ribose 2' and 4' form an oxymethylene bridge (a cyclic structure) by condensation . Therefore, the term "locked nucleic acid modification" or "LNA modification" in this application means that the nucleotide is modified to be a locked nucleic acid.

As used herein, the "deoxyribonucleotide substitution" modification refers to that the ribonucleotide in the RNA is replaced with its corresponding deoxyribonucleotide.

The term "2'-O-(2-methoxyethyl)" refers to a group formed by the substitution of the hydrogen atom at the second hydroxyl group on the ribose of the modified nucleotide by 2-methoxyethyl, and this process of the substitution by a 2-methoxyethyl is referred to as 2'-O-(2-methoxyethyl) modification.

Herein, when labeled in a nucleic acid sequence, "*" indicates a phosphorothioate-modified internucleotide linkage, "m" indicates that the nucleotide immediately to its left is modified by 2'-O-methylation, and "moe" indicates that the nucleotide immediately to its left is modified by 2'-O-(2-methoxyethyl), "+" indicates that the nucleotide immediately to its left is modified by LNA, "d " indicates that the nucleotide immediately to its left is substituted by a deoxyribonucleotide. As used herein, when ">" is used between two letters representing nucleotides, nucleosides or bases, it indicates a mutation in which the nucleotide represented by the letter to its left is modified to be the nucleotide represented by the letter to its right. For example, "G>A" indicates a mutation from guanosine to adenosine.

"Complementation" herein refers to the formation of hydrogen bonds between a nucleic acid and another nucleic acid through the traditional Watson-Crick base pairing principle. Complementation as used herein may refer to that the majority of nucleotides of two nucleic acids are complementary to each other, and not requiring all their nucleotides to be complementary.

"RNA editing" refers to a natural process present in eukaryotic cells, wherein an editing from base A (adenosine) to I (inosine) occurs at RNA level after DNA transcription and before protein translation, and during translation, inosine is recognized as G (guanosine), resulting in an A>G transition. This editing can occur through deamination of A to form I catalyzed by the ADAR (Adenosine Deaminase Acting on RNA) protease, ultimately producing targeted editing of a single nucleotide. Editing can occur in coding regions including intron and exon sequences, as well as in non-coding region sequences, and the editing of coding regions can redefine protein coding sequences.

As used herein, a "construct" refers to a nucleic acid comprising an RNA coding sequence or an amino acid coding sequence, wherein the coding sequence can express the RNA or amino acid by transcription or translation. The construct may be cyclic or linear, may be double-stranded or single-stranded, may be DNA or RNA, or a hybrid molecule of DNA and RNA, and may be chemically synthesized or biosynthetically synthesized. In some particular embodiments, the construct is a plasmid. In some particular embodiments, the construct is an oligonucleotide.

As used herein, "inrtoduce" or "introducting" into a cell refers to the process of making a protein or nucleic acid encoding the protein, RNA or a construct encoding the RNA, plasmid or other linear or circular DNA, etc., to pass through the cell membrane and enter the cell by transfection, infection, endocytosis, etc.

To address the current situation of lack of reliable therapies for Hurler syndrome, in the present application on the basis of the individualized features of Hurler syndrome, the mutant type (NM_000203.4(IDUA)-c.1205G>A (p.Trp402Ter)) with the highest percentage of IDUA causative genes is selected, and based on LEAPER technology, the length of the arRNA and the position of the targeting base are optimized, and at the same time appropriate chemical modifications are introduced to develop the functional RNA of the present application, i.e., arRNA, and the application methods thereof, for the treatment of Hurler syndrome. The arRNA can target and bind RNA sequences encoding α-L-iduronidase (IDUA) containing G>A mutation sites by complementary pairing of base sequences, recruiting deaminase to deaminate the adenosine (A) (target adenosine) of the mutation site to form an inosine (I), which will be recognized as G (guanosine) during translation, thereby realizing an A>G translation. The premature stop codon appeared due to the UGG>UAG mutation in the pre-mRNA or mRNA transcribed from the IDUA gene may be reverted to the codon UGG encoding tryptophan by using the arRNA and the method according to the present application, allowing the synthesis of IDUA enzyme to continue, and restoring the function of IDUA enzyme.

By constructing a reporter system with the IDUA pathogenic mutation (c. 1205G>A), while using GM06214 cells purchased from the Coriell Institute (Hurler syndrome patient-derived fibroblasts (TGG>TAG [Trp402Ter (W402X)] homozygous mutation at position 1293 of the IDUA gene in exon 9), and using GM01323 cells (Scheie syndrome patient-derived fibroblasts, containing a G>A transition (IVS5AS-7G>A) at position -7 of exon 6, i.e., intron 5) as a control, the function and characteristics of the arRNAs of this application were validated. The validation includes transfection of the arRNAs into cells containing the above-mentioned reporter system, or GM06214 cells, and the arRNA editing efficacy was tested by comparing the fluorescence intensity of the repoter system, the function of the IDUA enzyme, or by detecting the A>G editing efficiency or mutation rate by methods such as NGS (second generation sequencing technology).

### RNA editing method

In one aspect, the present application provides a method for targeted editing of a target RNA in a cell based on LEAPER technology, wherein the target RNA comprises a transcript sequence of an IDUA gene containing a guanosine (G) to adenosine (A) mutation site (target adenosine). The method comprises: introducing into the cell an arRNA or a construct comprising the arRNA coding sequence, wherein the arRNA comprises a complementary RNA sequence that hybridizes to the target RNA, and wherein the arRNA is capable of recruiting an adenosine deaminase acting on RNA (ADAR) by hybridization to the target RNA so as to deaminate the target adenosine in the target RNA.

In some embodiments, the 5' most adjacent nucleotide of the target adenosine in the target RNA is a nucleotide selected from U, C, A and G, and the order of preference is U> C ≈ A> G; and the 3' most adjacent nucleotide of the target adenosine in the target RNA is a nucleotide selected from G, C, A and U, and the order of preference is G> C> A ≈ U. In some embodiments, in a target RNA, the target adenosine is located in a target triplet selected from the group consisting of: UAG, UAC, UAA, UAU, CAG, CAC, CAA, CAU, AAG, AAC, AAA, AAU, GAG, GAC, GAA and GAU. In some embodiments, the target triplet formed by the target adenosine together with its 5' most adjacent nucleotide and 3' most adjacent nucleotide is 5'-UAG-3'. In some embodiments, the target RNA is selected from: pre-mRNA or mRNA transcribed from the IDUA gene. In some embodiments, the target RNA is a pre-mRNA transcribed from an IDUA gene.

In some embodiments, the arRNA comprises one or more mismatches, wobble pairings and/or unilateral bulges with complementary IDUA coding RNA sequences. In some embodiments, the arRNA does not comprise a region capable of forming an intramolecular stem-loop structure for recruiting an ADAR enzyme.

In some embodiments, the nucleotide in the arRNA opposite the target adenosine (targeting nucleotide) is a cytidine (C), an adenosine (A), or a uridine (U), preferably C. In some embodiments, the 5' most adjacent nucleotide of the targeting adenosine is a C, a G, or a U. In some embodiments, the 3' most adjacent nucleotide of the targeting adenosine is an A. In some embodiments, a targeting triplet is formed by the targeting nucleotide together with its 5' most adjacent nucleotide and 3' most adjacent nucleotide, wherein the targeting triplet is 5'-CCA-3'.

In some embodiments, the length of the arRNA is greater than 61nt, for example the length of the arRNAis 62-121nt, 62-111nt, 62-101nt, 62-100nt, 62-91nt, 62-76nt or 62-70nt; in some embodiments, the length of the arRNA is selected from any integer number of nucleotides of 62-121nt; in some particular embodiments, the length of the arRNAis 62nt, 63nt, 64t, 65nt, 66nt, 67nt, 68nt, 69nt, 70nt, 71nt, 72nt, 73nt, 74nt, 75nt, 76nt, 77nt, 78nt, 79nt, 80nt 81nt, 82nt, 83nt, 84nt, 85nt, 86nt, 87nt, 88nt, 89nt, 90nt, 92nt, 95nt, 98nt, 100nt, 103nt, or 106nt.

In some embodiments, the distance from the targeting nucleotide of the arRNA to the 3' end of the arRNAis less than the distance from the targeting nucleotide to the 5' end of the arRNA. In some embodiments, the distance from the targeting nucleotide of the arRNA to its 3' end is greater than 8nt, e.g., 9-60nt, preferably 9-45nt, or 9-35nt; in some embodiments, in order to avoid ultra-long poly G structures where possible, the distance from the targeting nucleotide of the arRNA to its 3' end is selected from 9-25nt, 9-26nt, or 9- 27nt; preferably, in some embodiments, the distance from the targeting nucleotide of the arRNA to its 3' end is selected from 9-20nt, 9-21nt, or 9-22nt; in some particular embodiments, the distance from the targeting nucleotide of the arRNA to its 3' end is any integer number of nucleotides selected from 9-25nt, e.g., 10nt, 11nt 12nt, 13nt, 14nt, 15nt, 16nt, 17nt, 18nt, 19nt, 23nt, or 24nt. In some embodiments, the distance from the targeting nucleotide of the arRNA to its 5' end is more than 25nt, e.g., 26-60nt, 35-60nt, or 45-60nt; in some preferred embodiments, the distance from the targeting nucleotide of the arRNA to its 5' end is selected from the group consisting of: 45nt, 45nt, 46nt, 47nt, 48nt, 49nt, 50nt, 51nt, 52nt, 53nt, 54nt, 55nt, 56nt, 57nt, 58nt, or 59nt.

In some embodiments, the distance from the targeting nucleotide of the arRNA to the 5' end of the arRNAis less than the distance from the targeting nucleotide to the 3' end of the arRNA. In some embodiments, the distance from the targeting nucleotide of the arRNA to the 5' end of the arRNA is greater than 35nt, e.g., 36-60nt, or 36-55nt; preferably, the distance from the targeting nucleotide of the arRNA to the 5' end of the arRNAis selected from the group consisting of: 54nt, 53nt, 52nt, 51nt, 50nt, 49nt, 48nt, 47nt, 46nt, 45nt, 44nt, 43nt, 42nt, 41nt, 40nt, 39nt, 38nt, or 36nt.

In some embodiments, the arRNA is an arRNA having a length charactorastic selected from the group consisting of: 55nt-c-35nt, 55nt-c-25nt, 55nt-c-24nt, 55nt-c-21nt, 55nt-c-20nt, 55nt-c-19nt, 55nt-c-18nt, 55nt-c-17nt, 55nt-c-16nt, 55nt-c-15nt, 55nt-c-14nt, 55nt-c-13nt, 55nt-c-12nt, 55nt-c-11nt, 55nt-c-10nt, 55nt-c-9nt, 50nt-c-20nt, 50nt-c-15nt, 45nt-c-55nt, or 45nt-c-20nt. Preferably, the arRNA is an arRNA having a length charactorastic selected from the group consisting of: 55nt-c-20nt, 55nt-c-15nt, or 55nt-c-14nt.

In some embodiments the arRNA is an oligonucleotide. In some embodiments the arRNA comprises one or more chemical modifications. It should be known to those of skill in the art that some appropriate chemical modifications can improve the editing efficiency of the arRNA by improving the stability of the RNA in the cell or *in vivo* while ensuring the editing efficiency, for example, adding 2'-O-methylation modifications to the nucleotides at both ends of the RNA, or making the internucleotide linkage undergo phosphorothioation modifications. As can be seen from the embodiments of the present application, the arRNA stability and editing efficiency fo the arRNA sequences according to the present application can be improved by one or more chemical modifications selected from: 2'-O-methylation modification, phosphorothioation modification, deoxyribonucleotide substitution modification, LNA modification, and 2'-O-(2-methoxyethyl) modification. Particularly, in some embodiments, the chemical modifications are one or more selected from the group consisting of:
1) 2'-O-methylation modifications in the first 2, 3, 4 or 5 nucleotides;
2) 2'-O-methylation modifications in the last 2, 3, 4, or 5 nucleotides;
3) 2'-O-methylation modifications in all the cytidines except that in the targeting triplet;
4) 2'-O-(2-methoxyethyl) modifications in the first 2, 3 or 4 nucleotides;
5) 2'-O-(2-methoxyethyl) modifications in the last 2, 3 or 4 nucleotides;
6) deoxyribonucleotide substitution modifications of the first 2, 3 or 4 nucleotides;
7) deoxyribonucleotide substitution modifications of the last 2, 3 or 4 nucleotides;
8) phosphorothioation modifications in the first 1, 2, 3, 4 or 5 internucleotide linkages;
9) phosphorothioation modifications in the last 1, 2, 3, 4 or 5 internucleotide linkages;
10) phosphorothioation modifications in all internucleotide linkages except that one or more of the 5' or 3' most adjacent internucleotide linkages of each of the nucleotides in the targeting triplet are not modified by phosphorothioation; and
11) phosphorothioation modifications in internucleotide linkages at an interval of 1, 2, 3, or 4 nucleotides except that one or more of the 5' or 3' most adjacent internucleotide linkages of each of the nucleotides in the targeting triplet are not modified by phosphorothioation.

Preferably, in some particular embodiments, the chemical modification is one or more selected from the group consisting of:
1) 2'-O-methylation modifications in the first 2, 3, 4 or 5 nucleotides and the last 2, 3, 4, or 5 nucleotides;
2) 2'-O-methylation modifications in all the cytidines except that in the targeting triplet;
3) 2'-O-(2-methoxyethyl) modifications in the first 2, 3 or 4 nucleotides and the last 2, 3 or 4 nucleotides;
4) deoxyribonucleotide substitution modifications of the first 2, 3 or 4 nucleotides and the last 2, 3 or 4 nucleotides;
5) phosphorothioation modifications in the first 1, 2, 3, 4 or 5 internucleotide linkages and the last 1, 2, 3, 4 or 5 internucleotide linkages;
6) phosphorothioation modifications in all internucleotide linkages, or at least every other internucleotide linkage, except that one or more of the 5' or 3' most adjacent internucleotide linkages of each of the nucleotides in the targeting triplet are not modified by phosphorothioation.

In some embodiments, the arRNA comprises any chemical modification selected from the group consisting of:
CM0, CM1, CM2, CM3, CM4, CM6, CM148, CM149, CM150, CM151, CM152, CM153, CM94, CM119, CM120, CM123, or CM125.

In some embodiments, the G to A mutation site targeted by the arRNA is a mutation site in NM_000203.4(IDUA)-c.1205G>A (p.Trp402Ter), or a corresponding mutation site thereof, in the genome of a patient with Hurler syndrome. In some embodiments, the arRNA comprises any sequence selected from the group consisting of:

1)Gm*Am*Cm-G*Cm-Cm*Cm-A*Cm-Cm*G-Um*G-Um*G-G*Um-Um*G-Cm*Um-G*Um-
Cm*Um-Cm*Cm-A*G-G*A-Cm*G-G*Um-Cm*G-G*Um-Cm*Cm-Cm*G-G*Cm-Cm*Um-G*Cm
-G*Cm-Cm*Um-G*Cm-G*Cm-G*A-Cm*A-Cm*Um-Um*Cm-G*G-Cm-C-C-A*G-A*G-Cm*Um-

G*Cm-Um*Cm-Cm*Um*Cm*Cm*Am (SEQ ID NO: 31);

2)Gm*Am*Cm-G*Cm-Cm*Cm-A*-Cm-Cm*G-Um*G-Um*G-G*Um-Um*G-Cm*Um-G*Um
-Cm*CM-A*G-G*A-Cm*G-G-G*Um-Cm*G*Cm*G-G*Um-Cm*Cm-Cm*G-G*Cm-Cm*Um-G*C
m-G*Cm*Um-G*-G*-A-Cm*A-Cm*A-Cm*Um-Um*Cm-G*G-Cm*Cd-Cd*Ad*-G-A*G-Cm*Um-
G*Cm-Um*Cm-Cm*Um*Cm*Am (SEQ ID NO: 31).

3)Gm*Am*Cm-G*Cm-Cm*Cm-A*Cm-Cm*G-Um*G-Um*G-G*Um-Um*G-G*Um-Um*G-C
m*Um-G*Um-Cm*Cm-A*G-G*A-Cm*-G-G*Um-Cm*-Cm*-G*Um-Cm*G-G*Cm-Cm*Um-G*G-
Cm*Um-G*-Cm*Um-G*-Cm-G*A-Cm*A-Cm*A-Cm*Um-Um*Cm-G*G-Cm-Cd-Cd*Ad*G-A*G-
Cm*Um-G*Cm-Um*Cm-Cm*Um*Cm*Am (SEQ ID NO: 31);

4)Gmoe*Amoe*Cmoe*Gmoe-C-C-C-A-C-C-C-G-Um-G-Um-G-G-Um-Um-G-C-Um-G-Um-C
-C-A-G-G-G-Um-G-Um-C-C-A-G-G-G-A-G-A-C-G-G-G-Um-C-C-G-Gm-G-C-C-C-A-G-A-G-C-U
m-G-C-Um-C*Cmoe*Umoe*Cmoe*Amoe (SEQ ID NO: 31); or

5)G+*A+*C+*G+C-C-C-A-C-C-C-G-Um-G-Um-G-G-Um-Um-G-C-Um-G-Um-C-C-A-G-G-
A-G-G-A-A-G-G-A-C-C-G-G-Um-G A-G-C-Um-G-C-Um-C*C+*U+*C+*A+ (SEQ ID NO: 31).

In some embodiments, the arRNA is introduced into the cell by a method selected from the group consisting of: electroporation, liposome transfection, exosome delivery, or lipid-nanoparticle delivery (LNP). Preferably, the arRNA is introduced into the cell by liposome transfection. In some embodiments, the cell is a human cell. In some embodiments, the cell is selected from the group consisting of: stem cells, chondrocytes, periosteal cells, mesenchymal cells, cardiomyocytes, neural cells, or hepatocytes. In some embodiments, the cell is preferably a hepatocyte.

In some embodiments, the method for targeted editing of the target RNA in a cell based on LEAPER technology comprises introducing into the cell a plurality, e.g., 2, 3, 4 or more, of arRNAs with different sequences and/or having different chemical modifications.

In some embodiments, the arRNAs are introduced into the cells by multiple times, e.g., 2, 3, 4, or more times. In some embodiments, the method used for each introduction of arRNA is the same, e.g., all are liposome transfection, or all are lipid-nanoparticle delivery. In some embodiments, the method used for each introduction of the arRNAs is different depending on the state of the cell.

In some embodiments, the cells are introduced with the arRNA for only once. in some embodiments, the amount of arRNA in a single introduction into the cells is ≥5nM, preferably ≥ 10nM, further preferably ≥20nM; in some particular embodiments, the amount of arRNA in a single introduction into the cells is 5-160nM, e.g., 10-160nM, 20-160nM 40-160nM, 20-80nM, 40-80nM, or 20-40nM; in some particular embodiments, the amount of arRNA in a single introduction into the cells is 15nM, 17nM, 19nM, 21nM, 22nM, 23nM, 24nM, 25nM, 26nM, 27nM, 28nM, 29nM, 30nM, 31nM, 32nM, 33nM, 34nM, 35nM, 36nM, 37nM, 38nM, 39nM, 41nM, 43nM, 45nM, 55nM, 65nM, 75nM, 85nM, 95nM, 105nM, 115nM, 125nM, 135nM, 145nM, or 155nM. In some embodiments, several introductions are taken for introducing the arRNA into the cells, and the interval between the adjacent two introductions is ≤ 21 days, e.g., >_17 days, >_14 days, >_12 days, >_11 days, or ≥10 days; in some embodiments, the interval between the adjacent two introductions is 9-20 days, 9-16 days, 9-14 days, 12-19 days, 12-15 days, or 12- 13 days; in some embodiments, the interval between the adjacent two introductions is 7 days, 8 days, 11 days, 15 days, 18 days, 19 days, or 20 days.

In some embodiments, the method for targeted editing of the target RNA in the cell based on LEAPER technology does not include introducing any exogenous protein or coding sequence of an exogenous protein into the cell. In some embodiments, the method further comprises introducing an exogenous ADAR into the patient cell. In some particular embodiments, the exogenous ADAR is ADAR1 comprising the E1008 mutation. In some embodiments, the method may also comprise introducing an inhibitor of ADAR3 into the host cell. In some embodiments, the method further comprises introducing a stimulator of an interferon into the host cell.

As can be seen from the data and results of the examples of this application, according to the method described in this application the recovery rate (A>G editing efficiency) of the target adenosine (G>A) mutation site is at least about 20%, e.g., at least about 25%, 30%, 35%, 40%, 45%, 50%, 55%, 60%, 65%, 70%, 75%, 80%, 85%, 90% or higher.

### arRNA

In one aspect, the present application further provides an arRNA comprising a complementary RNA sequence that hybridizes to a target RNA, and wherein the arRNA is capable of recruiting an adenosine deaminase acting on RNA (ADAR) by hybridization to the target RNA so as to deaminate the target adenosine in the target RNA, wherein the target RNA comprises a guanosine (G) to adenosine (A) mutation site (target adenosine). In some embodiments, a target triplet is formed by the target adenosine together with its 5' most adjacent nucleotide and its 3' most adjacent nucleotide, wherein the target triplet is 5'-UAG-3'.

In some embodiments, the arRNA comprises one or more mismatches, wobble pairings and/or unilateral bulges with the complementary IDUA encoding RNA sequence. In some embodiments, the arRNA does not comprise a region capable of forming an intramolecular stem-loop structure for recruiting an ADAR enzyme. In some embodiments, the nucleotide in the arRNA opposite the target adenosine (targeting nucleotide) is a cytidine (C), an adenosine (A), or a uridine (U), preferably a C. In some embodiments, the 5' most adjacent nucleotide of the targeting adenosine is a C, a G, or a U. In some embodiments, the 3' most adjacent nucleotide of the targeting adenosine is an A. In some embodiments, a targeting triplet formed by the targeting nucleotide togetner with its 5' most adjacent nucleotide and 3' most adjacent nucleotide is 5'-CCA-3'. In some embodiments, the arRNA further comprises one or more guanosines, each of which is opposite a particular adenosine (non-target adenosine) other than the target adenosine in the target RNA. In some embodiments, the arRNA comprises two or more contiguously mismatched nucleotides opposite non-target adenosines in the target RNA.

In some embodiments the length of the arRNA is greater than 61nt, for example the length of the arRNAis 62-121nt, 62-111nt, 62-101nt, 62-100nt, 62-91nt, 62-76nt, or 62-70nt; in some embodiments the length of the arRNA is an integer number of nucleotides selected from any of 62-121nt; in some particular embodiments the length of the arRNA is 62nt, 63nt, 64t, 65nt, 66nt, 67nt, 68nt, 69nt, 70nt, 71nt, 72nt, 73nt, 74nt, 75nt, 76nt, 77nt, 78nt, 79nt, 80nt 81nt, 82nt, 83nt, 84nt, 85nt, 86nt, 87nt, 88nt, 89nt, 90nt, 92nt, 95nt, 98nt, 100nt, 103nt, or 106nt.

In some embodiments, the distance from the targeting nucleotide of the arRNA to the 3' end of the arRNA is less than the distance from the targeting nucleotide to the 5' end of the arRNA. In some embodiments, the distance from the targeting nucleotide of the arRNA to its 3' end is greater than 8nt, e.g., 9-60nt, preferably 9-45nt, or 9-35nt; in some embodiments, in order to avoid ultra-long poly G structures where possible, the distance from the targeting nucleotide of the arRNA to its 3' end is selected from: 9-25nt, 9-26nt, or 9-27nt; preferably, in some embodiments, the distance from the targeting nucleotide of the arRNA to its 3' end is selected from the group: 9-20nt, 9-21nt, or 9-22nt; in some particular embodiments, the distance from the targeting nucleotide of the arRNA to its 3' end is any integer number of nucleotides selected from 9-25nt, e.g., 10nt, 11nt 12nt, 13nt, 14nt, 15nt, 16nt, 17nt, 18nt, 19nt, 23nt, or 24nt. In some embodiments, the distance from the targeting nucleotide of the arRNA to its 5' end is greater than 25nt, e.g., 26-60nt, 35-60nt, or 45-60nt; in some preferred embodiments, the distance from the targeting nucleotide of the arRNA to its 5' end is selected from the group consisting of: 45nt, 45nt, 46nt, 47nt, 48nt, 49nt, 50nt, 51nt, 52nt, 53nt, 54nt, 55nt, 56nt, 57nt, 58nt, or 59nt.

In some embodiments, the distance from the targeting nucleotide of the arRNA to the 5' end of the arRNA is less than the distance from the targeting nucleotide to the 3' end of the arRNA. In some embodiments, the distance from the targeting nucleotide of the arRNA to the 5' end of the arRNA is greater than 35nt, e.g., 36-60nt, or 36-55nt; preferably, the distance from the targeting nucleotide of the arRNA to the 5' end of the arRNA is selected from the group consisting of: 54nt, 53nt, 52nt, 51nt, 50nt, 49nt, 48nt, 47nt, 46nt, 45nt, 44nt, 43nt, 42nt, 41nt, 40nt, 39nt, 38nt, or 36nt.

In some embodiments, the arRNA is an arRNA having a length charactorastic selected from the group consisting of: 55nt-c-35nt, 55nt-c-25nt, 55nt-c-24nt, 55nt-c-21nt, 55nt-c-20nt, 55nt-c-19nt, 55nt-c-18nt, 55nt-c-17nt 55nt-c-16nt, 55nt-c-15nt, 55nt-c-14nt, 55nt-c-13nt, 55nt-c-12nt, 55nt-c-11nt, 55nt-c-10nt, 55nt-c-9nt, 50nt-c-20nt, 50nt-c-15nt, 45nt-c-55nt, or 45nt-c-20nt. Preferably, the arRNA is an arRNA having a length charactorastic selected from the group consisting of: 55nt-c-20nt, 55nt-c-15nt, or 55nt-c-14nt.

In some embodiments the arRNA is an oligonucleotide. In some embodiments the arRNA comprises one or more chemical modifications. In some embodiments the chemical modification is one or more selected from the group consisting of: 2'-O-methylation modification, phosphorothioation modification, deoxyribonucleotide substitution modification, LNA modification, and 2'-O-(2-methoxyethyl) modification. In some embodiments, the chemical modification is one or more selected from the group consisting of:
1) 2'-O-methylation modifications in the first 2, 3, 4 or 5 nucleotides;
2) 2'-O-methylation modifications in the last 2, 3, 4, or 5 nucleotides;
3) 2'-O-methylation modifications in all the cytidines except that in the targeting triplet;
4) 2'-O-(2-methoxyethyl) modifications in the first 2, 3 or 4 nucleotides;
5) 2'-O-(2-methoxyethyl) modifications in the last 2, 3 or 4 nucleotides;
6) deoxyribonucleotide substitution modifications of the first 2, 3 or 4 nucleotides;
7) deoxyribonucleotide substitution modifications of the last 2, 3 or 4 nucleotides;
8) phosphorothioation modifications in the first 1, 2, 3, 4 or 5 internucleotide linkages;
9) phosphorothioation modifications in the last 1, 2, 3, 4 or 5 internucleotide linkages;
10) phosphorothioation modifications in all internucleotide linkages except that one or more of the 5' or 3' most adjacent internucleotide linkages of each of the nucleotides in the targeting triplet are not modified by phosphorothioation; and
11) phosphorothioation modifications in internucleotide linkages at an interval of 1, 2, 3, or 4 nucleotides except that one or more of the 5' or 3' most adjacent internucleotide linkages of each of the nucleotides in the targeting triplet are not modified by phosphorothioation.

Preferably, in some particular embodiments the chemical modification is one or more selected from the group consisting of:
1) 2'-O-methylation modifications in the first 2, 3, 4 or 5 nucleotides and the last 2, 3, 4, or 5 nucleotides;
2) 2'-O-methylation modifications in all the cytidines except that in the targeting triplet;
3) 2'-O-(2-methoxyethyl) modifications in the first 2, 3 or 4 nucleotides and the last 2, 3 or 4 nucleotides;
4) deoxyribonucleotide substitution modifications of the first 2, 3 or 4 nucleotides and the last 2, 3 or 4 nucleotides;
5) phosphorothioation modifications in the first 1, 2, 3, 4 or 5 internucleotide linkages and the last 1, 2, 3, 4 or 5 internucleotide linkages;
6) phosphorothioation modifications in all internucleotide linkages, or at least every other internucleotide linkage, except that one or more of the 5' or 3' most adjacent internucleotide linkages of each of the nucleotides in the targeting triplet are not modified by phosphorothioation.

In some embodiments, the arRNA comprises any chemical modification selected from the group consisting of:
CM0, CM1, CM2, CM3, CM4, CM6, CM148, CM149, CM150, CM151, CM152, CM153, CM94, CM119, CM120, CM123, or CM125.

In some embodiments, the target RNA targeted by the arRNA is selected from: pre-mRNA, mature mRNA, ribosomal RNA, transfer RNA, long-stranded non-coding RNA, or small RNA. In some embodiments, the target RNA is a pre-messenger RNA. In some embodiments, the target RNA is a transcript sequence of an IDUA gene. In some embodiments, the target RNA is a pre-mRNA or mature mRNAtranscribed from the IDUA gene. In some embodiments, the target RNAis a pre-mRNA transcribed from the IDUA gene. In some embodiments, the target RNA comprises a mutation site equivalent to NM_000203.4(IDUA)-c.1205G>A (p.Trp402Ter) mutation site in the genome of a patient with Hurler syndrome, and when the arRNA hybridizes to the target RNA, the targeting nucleotide of the arRNA is opposite to the mutation site in the target RNA. Meanwhile, the mutation site is the target nucleotide of the arRNA. In some embodiments, the arRNA may be used in the RNA editing method previously described.

In some embodiments, the arRNA comprises any sequence selected from the group consisting of:

1)Gm*Am*Cm-G*Cm-Cm*Cm-A*Cm-Cm*G-Um*G-Um*G-G*Um-Um*G-Cm*Um-G*Um-
Cm*Um-Cm*Cm-A*G-G*A-Cm*G-G*Um-Cm*G-G*Um-Cm*Cm-Cm*G-G*Cm-Cm*Um-G*Cm
-G*Cm-Cm*Um-G*Cm-G*Cm-G*A-Cm*A-Cm*Um-Um*Cm-G*G-Cm-C-C-A*G-A*G-Cm*Um-
G*Cm-Um*Cm-Cm*Um*Cm*Cm*Am (SEQ ID NO: 31);

2)Gm*Am*Cm-G*Cm-Cm*Cm-A*-Cm-Cm*G-Um*G-Um*G-G*Um-Um*G-Cm*Um-G*Um
-Cm*CM-A*G-G*A-Cm*G-G-G*Um-Cm*G*Cm*G-G*Um-Cm*Cm-Cm*G-G*Cm-Cm*Um-G*C
m-G*Cm*Um-G*-G*-A-Cm*A-Cm*A-Cm*Um-Um*Cm-G*G-Cm*Cd-Cd*Ad*-G-A*G-Cm*Um-
G*Cm-Um*Cm-Cm*Um*Cm*Am (SEQ ID NO: 31);

3)Gm*Am*Cm-G*Cm-Cm*Cm-A*Cm-Cm*G-Um*G-Um*G-G*Um-Um*G-G*Um-Um*G-C
m*Um-G*Um-Cm*Cm-A*G-G*A-Cm*-G-G*Um-Cm*-Cm*-G*Um-Cm*G-G*Cm-Cm*Um-G*G-
Cm*Um-G*-Cm*Um-G*-Cm-G*A-Cm*A-Cm*A-Cm*Um-Um*Cm-G*G-Cm-Cd-Cd*Ad*G-A*G-
Cm*Um-G*Cm-Um*Cm-Cm*Um*Cm*Am (SEQ ID NO: 31);

4)Gmoe*Amoe*Cmoe*Gmoe-C-C-C-A-C-C-C-G-Um-G-Um-G-G-Um-Um-G-C-Um-G-Um-C
-C-A-G-G-G-Um-G-Um-C-C-A-G-G-G-A-G-A-C-G-G-G-Um-C-C-G-Gm-G-C-C-C-A-G-A-G-C-U
m-G-C-Um-C*Cmoe*Umoe*Cmoe*Amoe (SEQ ID NO: 31); or

5)G+*A+*C+*G+C-C-C-A-C-C-C-G-Um-G-Um-G-G-Um-Um-G-C-Um-G-Um-C-C-A-G-G-
A-G-G-A-A-G-G-A-C-C-G-G-Um-G A-G-C-Um-G-C-Um-C*C+*U+*C+*A+ (SEQ ID NO: 31).

### Construct, Composition, Particle, Cell, Library or Kit

In one aspect, the present application also provides a construct comprising a coding sequence of the arRNA previously described herein. In some embodiments, the construct is a single-stranded DNA. In some embodiments, the construct is a double-stranded DNA. In some embodiments, the construct is a hybrid molecule of RNA and DNA. In some embodiments, the construct is a double-stranded RNA. In some embodiments, the construct comprises an antisense nucleic acid sequence of the arRNA.

In some embodiments, the construct is in the form of a loop. In some embodiments, the construct is linear.

In some embodiments, the construct is a plasmid. In some embodiments, the construct comprises all or part of the genome of a virus. In some embodiments, the virus is selected from AAV, lentivirus, phage, or baculovirus. Thus, the present application also provides a virus comprising the construct. In one aspect, the present application also provides a compositions, a liposome, an exosome, a lipid-nanoparticle, a cell, a library, or a kit comprising an aforementioned arRNA or an aforementioned construct. In some embodiments, the composition, liposome, exosome, lipid-nanoparticle, cell, library, or kit comprises only one of the aforementioned arRNAs or constructs comprising the arRNA coding sequences. In some embodiments, the composition, liposome, exosome, lipid-nanoparticle, cell, library, or kit comprises two or more of the aforementioned arRNAs or constructs comprising the arRNA coding sequences.

In some embodiments, the composition is a pharmaceutical composition, so that the composition further comprises one or more pharmaceutically acceptable excipients.

In some embodiments, the cell comprises the construct, and the construct is located in the cytoplasm. In some embodiments, the cell comprises the construct, and the construct is a part of the genomic DNA.

### Method for preventing or treating Hurler syndrome

In one aspect, the present application also provides a method for treating or preventing Hurler syndrome, the method comprises administering to a patient (with Hurler syndrome having a mutation site equivalent to NM_000203.4(IDUA)-c.1205G>A (p.Trp402Ter)) an aforementioned arRNA, or a aforementioned construct, liposome, exosome, lipid-nanoparticle or cell, or preventing or treating the symptoms associated with Hurler syndrome in a patient by the aforementioned RNA editing method.

In some embodiments, the administration is selected from: intravenous injection, subcutaneous injection, arterial perfusion, intramuscular injection, single organ local injection, or intracranial injection. In some embodiments, the administration is a single administration, for example a single infusion of cells comprising the arRNA coding sequence to the patient, thereby allowing the cells to colonize in a particular tissue.

In some embodiments, the administration is performed 2, 3, or more times. In some embodiments, the interval between two adjacent administrations is longer than 21 days. In some embodiments, the interval between two adjacent administrations is ≥ 17 days, >_ 14 days, >_ 12 days, >_ 11 days, or ≥ 10 days. In some embodiments, the interval between the two adjacent introductions is 9-20 days, 9-16 days, 9-14 days, 12-19 days, 12-15 days, or 12-13 days. In some embodiments, the interval between two adjacent introductions is 7 days, 8 days, 11 days, 15 days, 18 days, 19 days, 20 days.

In some embodiments, the administration maintains arRNA concentration in the target tissue at ≥5nM, preferably ≥10nM, further preferably ≥20nM. In some particular embodiments, the administration maintains arRNA concentration in the target tissue at 5-160nM, e.g., 10-160nM, 20-160nM, 40-160nM, 20 -80nM, 40-80nM, or 20-40nM. In some particular embodiments, the administration maintains arRNA concentration in the target tissue at 15nM, 17nM, 19nM, 21nM, 22nM, 23nM, 24nM, 25nM, 26nM, 27nM, 28nM, 29nM, 30nM, 31nM, 32nM , 33nM, 34nM, 35nM, 36nM, 37nM, 38nM, 39nM, 41nM, 43nM, 45nM, 55nM, 65nM, 75nM, 85nM, 95nM, 105nM, 115nM, 125nM, 135nM, 145nM, or 155nM.

The technical solutions of the present application are further described in detail below with reference to the following specific examples, but the application is not limited to these examples. If not specifically noted, the reagents involved below are commercially available. For the sake of simplicity, some of the operations are not detailed in terms of the parameters, the steps and the apparatus used in the operations, it should be understood that these are well known and reproducible by those skilled in the art.

### EXAMPLES

The cells, GM06214 and GM01323, used herein were purchased from Coriell, USA, wherein GM06214 was a fibroblast derived from a patient with Hurler syndrome with a mutation from G to A at nucleotide 1293 in exon 9 of the IDUA gene, and the codon of which was mutated from TGG to TAG, ([Trp402Ter (W402X)]) (the above description of the cell was from the product specification, wherein the mutation described corresponds to NM_000203.4(IDUA)-c.1205G>A (p.Trp402Ter) mutation). While GM01323 was a fibroblast derived from a patient with Scheie syndrome (SDS), whose IDUA activity was 0.3% of that in healthy human-derived fibroblasts. Compared to Hurler syndrome, the symptoms of Scheie syndrome are relatively mild. Therefore, GM01323 was used as a positive control of IDUA enzyme activity, and GM06214 was used as a disease model and test cell in the following examples.

### Example 1: Detection of Genotype of GM06214 Mutant

GM06214 cells were placed in fibroblast culture medium (ScienCell, FM medium, Catalog. No. 2301) containing 15% serum and added with 1% fibroblast growth supplement (ScienCell, GFS, Catalog. No. 2301 ), incubating for 2-3 days at 37°C in a 5% CO₂ incubator. When cell confluence reached 90%, the cells were digested with 0.25% trypsin, then the digestion was terminated by using a fibroblast culture medium containing 15% serum. DNA extraction was performed by using the TianGene^{®} (TIANGEN Biotech (Beijing) Co., Ltd.) Cell DNA Extraction Kit (Catalog. No. DP304-03) according to the operating instructions.

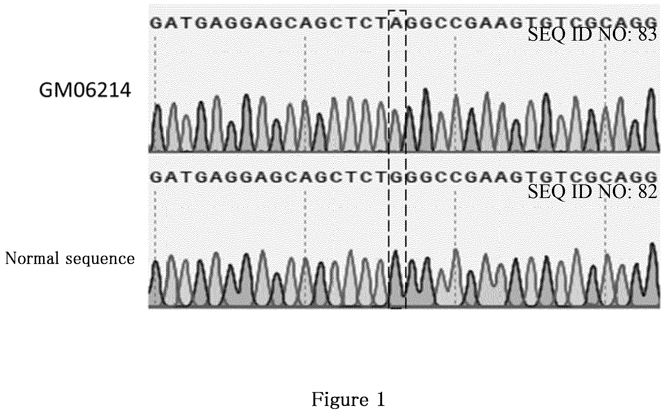

Primer design for the upstream and downstream sequences of IDUA target sites was performed by using NCBI-Primer blast (URL: https://www.ncbi.nlm.nih.gov/tools/primer-blast/). SEQ ID NO: 1: CGCTTCCAGGTCAACAACAC (forward primer hIDUA-F1); SEQ ID NO: 2: CTCGCGTAGATCAGCACCG (reverse primer hIDUA-R1). PCR reactions were performed, and PCR products were subjected to sanger sequencing. As shown in Fig. 1, it was verified that the mutant type of the above GM06214 cell was confirmed as a pathogenic mutation of G to A at position 15704 on the IDUA genome, i.e.: c.1205G>A (p.Trp402Ter) mutation.

### Example 2: Detection of IDUA Enzyme Activity and Editing Efficiency after Electroporation of GM06214 Cells with arRNA

The following arRNA sequences were designed and synthesized for the upstream and downstream sequences of the target sites in the mRNA precursor (pre-mRNA) and the mature mRNA after transcription of the IDUA gene:
Targeting arRNA for the pre-mRNA: 
GACGCCCACCGUGUGGUUGCUGUCCAGGACGGUCCCGGCCUGCGACACUUCGGCCCA
GAGCUGCUCCUCAUCCAGCAGCGCCAGCAGCCCCAUGGCCGUGAGCACCGGCUU (SEQ
ID NO: 3, Pre-55nt-c-55nt).
Targeting arRNA for the mature mRNA: GACGCCCACCGUGUGGUUGCUGUCCAGGACGGUCCCGGCCUGCGACACUUCGGCCCA 
GAGCUGCUCCUCAUCUGCGGGCGGGGGGGCCGUCGCCGCGUGGGGUCGUUG
( SEQ ID NO: 4, m-55nt-c-55nt).
Randomly designed non-targeting arRNA:

UACCGCUACAGCCACGCUGAUUUCAGCUAUACCUGCCCGGUAUAAAGGGACGUUCAC
ACCGCGAUGUUCUCUGCUGGGGAAUUGCGCGAUAUUCAGGAUUAAAAGAAGUGC
(SEQ ID NO: 5, Random-111nt);

Particularly, the bases in the arRNAs targeting the mutation sites (target adenosine of the target sequence) of the mRNA precursor (pre-mRNA) and the mature mRNA was C, such that an A-C mismatch was formed between the target adenosine and the base in the arRNA corresponding to the target adenosine. The length of the synthesized arRNA was chosen to be 111 nt. The synthesized arRNA was chemically modified with the pattern of CM0 (the first 3 and last 3 nucleotides in the arRNA sequence were modified by 2'-O-methylation, and the first 3 and last 3 internucleotide linkages were modified by phosphorothioation modification, respectively). Cells were electroporated by the following steps.

The cells were digested when the GM06214 cells grew to approximately 90% confluence, then terminating the digestion and counting. For electroporation, 6 million cells were resuspended with 400 µl of premixed electroporation solution (Lonza, Catalog. No. V4XP-3024), then adding 20 µg of arRNA to mix well, taking each 20 µl as one electroporation system, and performing electroporation by using the preset electroporation conditions of the Lonza Nucleofector electroporation instrument. After electroporation, the cells were rapidly transferred into 2 ml of fibroblast culture medium (ScienCell, FM medium, Catalog. No. 2301) containing 15% serum, and inoculated in 6-well plates. The cells were incubated in a 37°C, 5% CO₂ incubator.

Forty-eight hours after electroporation, cells were collected and assayed for enzyme activity and A to G mutation rate in IDUA mRNA.

### Detection of A to G mutation rate in IDUAmRNA

The designed synthetic arRNA was dissolved to the desired concentration with RNAase-free water (Transgen Biotech, Catalog. No. GI201-01,) and stored at -80°C. Cells were digested when GM06214 cells grew to approximately 90% confluence, counting after termination of the digestion. One million cells were taken to add 200 pmol of arRNA, and electroporation was performed in a volume of 100 µl. Forty-eight hours after electroporation, the cells were counted and the viability was measured. The cells were transferred into RNAase-free centrifuge tubes, and the supernatant was discarded, and RNA was extracted by using the QIAGEN RNA extraction kit (QIAGEN, Catalog. No. 74134). According to the instructions, 0.35 ml of Buffer RLT Plus was added as per 5 × 10⁵ cells to mix by pipetting up and down (if frozen cells were derectly used for RNA extraction, washing once with PBS). Suspension of lysed cells was added to a gDNA Eliminator centrifuge column, centrifuging at ≥8000g for 30s, then discarding the column to leave the liquid. 70% ethanol of the volume was added to mix by pippeting up and down, then immediately performing the next step. The liquid was added to the RNeasyMinElute centrifuge column centrifuging at ≥8000g for 15s, then discarding the waste liquid. 700 µl of Buffer RW1 was added to the RNeasyMinElute centrifuge column, centrifuging at ≥8000g for 15 s, then discarding the waste solution. 500 µl of Buffer RPE was added to the RNeasyMinElute column, centrifuging at ≥8000g for 15s, then discarding the waste liquid. 500 µl of 80% ethanol was added to the RNeasyMinElute centrifuge column, centrifuging at ≥8000g for 2 min, then discarding the waste liquid. The RNeasyMinElute centrifuge column was placed into a 2 ml collection tube, centrifuging at maximum speed with the cap open for 5 min to allow the column to dry. The RNeasyMinElute centrifuge column was placed into a 1.5 ml collection tube, and 14 µl of RNAase-free water was added dropwise at the center of the centrifuge column membrane, then centrifuging at maximum speed for 1 min to elute RNA.

The concentration of the extracted RNA was measured with Nanodrop (Thermo, Catalog. No. Nanodrop2000), and 1 µg RNA was used for reverse transcription (Thermo, reverse transcriptase Catalog. No. 28025013). The reverse transcription system was configured as in Table 1, after incubating at 65°C for 5 min, it is immediately cooled in an ice bath. Then the system as shown in Table 2 was used to incubate continuously at 37°C for 50 minutes. The reverse transcriptase was then inactivated at 70°C for 15 minutes. PCR was performed by using the PCR primers in Example 1 under the conditions shown in Table 3. After PCR, 2 µl of PCR product was taken for agarose gel electrophoresis, and the concentration of the PCR product and the correct band size were initially determined based on the electrophoresis results. After purification, the PCR product was used for library preparation, and sent for second-generation sequencing.

All subsequent tests involving reverse transcription were performed by using the reverse transcription system and procedures in this example.

**Table 1. Configuration-1 of the reverse transcription system**

| | Volume (µl) |
|---|---|
| Total RNA(1µg) | X |
| Oligo dT | 1 |
| 10nM dNTP | 1 |
| RNase free water | 10-X |
| Total volume | 12 |

**Table 2. Configuration-2 of the reverse transcription system**

| | Volume (µl) |
|---|---|
| System of table 1 | 12µl |
| 5X First-Strand buffer | 4 |
| 0.1MDTT | 2 |
| RNaseOUT^{™} recombinant nuclease inhibitor | 1 |
| M-MLV | 1 |
| Total volume | 20 |

**Table 3. PCR conditions**

| Steps | Time | Cycle |
|---|---|---|
| 98°C | 2min | 1 cycle |
| 98°C | 15s | 28-35 cycles |
| 63 °C | 30s | |
| 72°C | 15s | |
| 72°C | 2min | 1 cycle |

### Enzyme activity detection

After digestion, the GM06214 cells were centrifuged, then resuspending in 28 µl of 1× PBS containing 0.1% Triton X-100, and lysing on ice for 30 min. Then 25 µl of cell lysate was added to 25 µl of substrate containing 190 µm 4-methylumbelliferyl-α-L-iduronidase (Cayman, 2A-19543-500, dissolved in 0.4 M sodium formate buffer containing 0.2% Triton X-100, pH 3.5), incubating for 90 min at 37°C in the dark. 200 µl of 0.5 M NaOH/glycine solution (Beijing Chemical, NAOH, Catalog. No. AR500G; Solarbio, Glycine Catalog. No.G8200) with PH=10.3 was added to inactivate the catalytic reaction, centrifuging at 4°C for 2 min. The supernatant was transferred to 96-well plates, and the fluorescence values were measured on an Infinite M200 instrument (TECAN) with 365 nm and 450 nm excitation wavelengths.

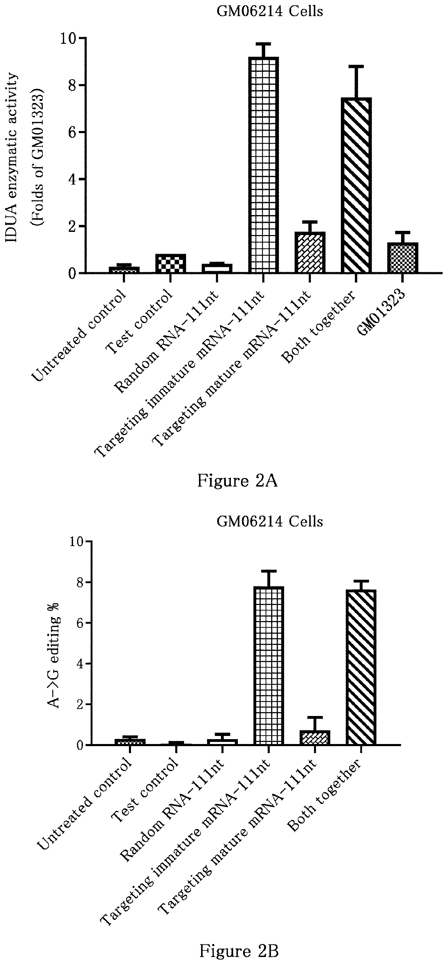

The results of this example were shown in Fig. 2, when targeting pre-mRNA, arRNA was able to exhibit high enzyme activity and editing efficiency, while targeting mature mRNA the arRNA exhibited significantly lower enzyme activity and editing efficiency. Therefore, the arRNAs involved in the later examples were all arRNAs targeting pre-mRNA.

### Example 3: Detection of Editing Efficiency of IDUA Target Site on IDUA-reporter Cell Line after Electroporation of arRNA

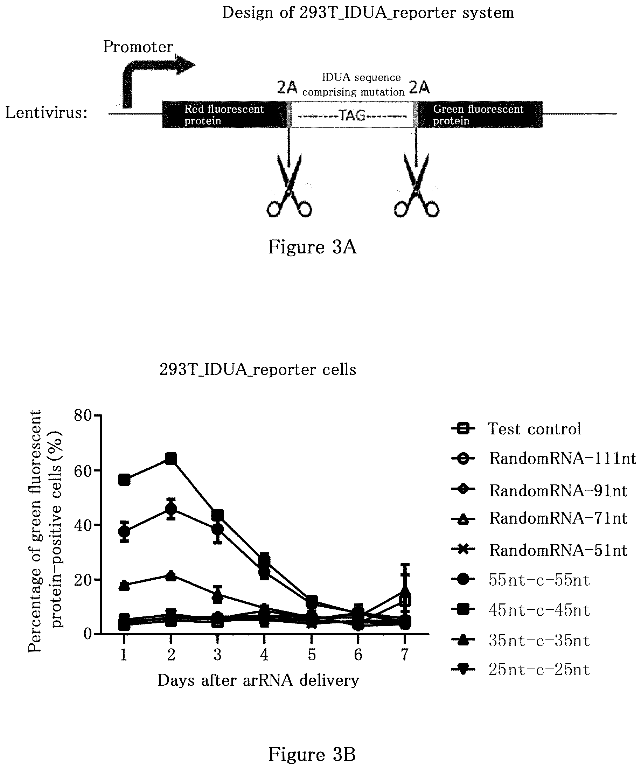

As shown in Fig. 3A, an IDUA gene transcript sequence segment containing the human (NM_000203.4(IDUA)-c.1205G>A (p.Trp402Ter))(IDUA)_c. 1239 G>A (p.Trp402Ter) mutation site and about 100bp of each upstream and downstream was inserted between the expressing sequences of mCherry and GFP proteins on the lentiviral plasmid to construct the plasmid. The above constructed plasmid was packaged as viruses to infect 293T cells. IDUA-reporter monoclonal cells were screened out after integration of the virus into the genome. The monoclonal cells expressed only mcherry protein due to affecting by the TAG stop (where the IDUA target adenosine was located) codon in the inserted sequence, and when the cell was edited by arRNA, it was converted from TAG to TGG, then the subsequent GFP protein was expressed normally. The ratio of positive cells for GFP protein may reflect the editing efficiency of arRNA-edited cells. We preferably designed four (25nt-c-25nt, 35nt-c-35nt, 45nt-c-45nt, and 55nt-c-55nt) arRNAs of different lengths from 51nt to 111nt, and four non-targeting random control arRNAs (111nt-random, 91nt-random, 71nt- random, and 51nt-random). Each arRNA was chemically modified with the CM0 pattern. The arRNA sequences used in this Example were shown in Table 4 below.

After cells were separately transfected with different arRNAs with the electroporation conditions in Example 2, the ratio of GFP-positive cells was detected from day 1 to day 7 respectively, for a preliminary comparison of editing efficiency. As seen in Fig. 3B, the highest peak of editing occurred on day 2 (48h). The sequence with the highest editing efficiency was 45nt-c-45nt with a total length of 91nt, and the arRNA with a total length of 51nt had a very low editing efficiency. This shows that the length of the arRNA should not be too short, but also it was not the case that the longer the sequence the higher the editing efficiency.

**Table 4.**

| | |
|---|---|
| 111nt-random | SEQ ID NO: 5 |
| | uaccgcuacagccacgcugauuucagcuauaccugcccgguauaaagggacguucacaccgcg auguucucugcuggggaauugcgcgauauucaggauuaaaagaagugc |
| 91nt-random | SEQ ID NO: 6 uaauccugaauaucgcgcaauuccccagcagagaacaucgcggugugaacgucccuuuauacc gggcagguauagcugaaaucagcguggc |
| 71nt-random | SEQ ID NO: 7 uuucagcuauaccugcccgguauaaagggacguucacaccgcgauguucucugcuggggaau ugcgcgaua |
| 51nt-random | SEQ ID NO: 8 uuccccagcagagaacaucgcggugugaacgucccuuuauaccgggcaggu |
| 55nt-c-55nt | SEQ ID NO: 4 gacgcccaccgugugguugcuguccaggacggucccggccugcgacacuucggcccagagcug cuccucaucugcggggcgggggggggccgucgccgcguggggucguug |
| 45nt-c-45nt | SEQ ID NO: 9 gugugguugcuguccaggacggucccggccugcgacacuucggcccagagcugcuccucauc ugcggggcgggggggggccgucgccgcgu |
| 35nt-c-35nt | SEQ ID NO: 10 uguccaggacggucccggccugcgacacuucggcccagagcugcuccucaucugcggggcgg gggggggcc |
| 25nt-c-25nt | SEQ ID NO: 11 ggucccggccugcgacacuucggcccagagcugcuccucaucugcggggcg |

### Example 4: Detection of RNA Editing Efficiency at Different Time Points after ELectroporation of GM06214 Cells with Different Lengths of arRNA

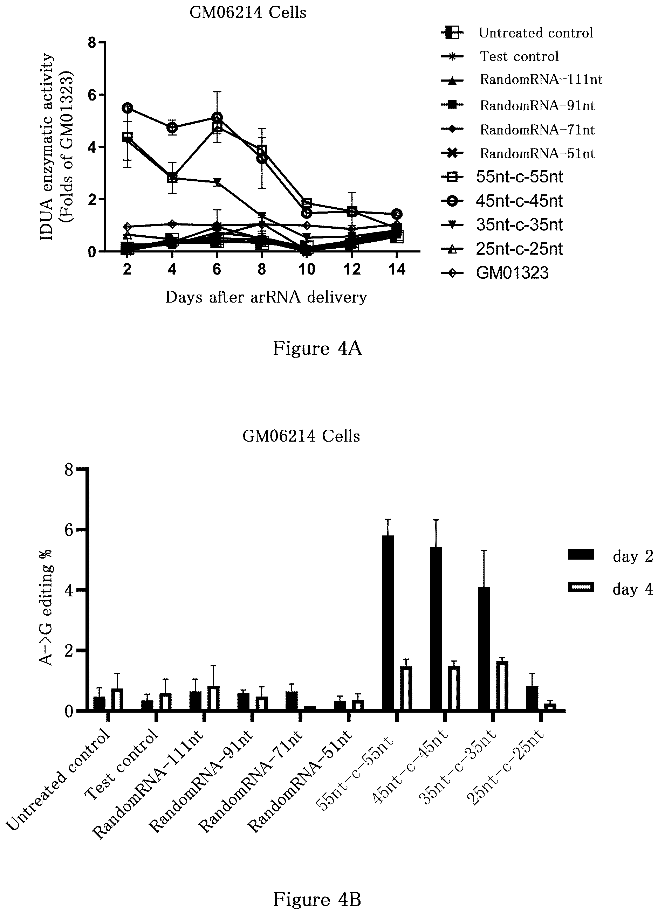

Different lengths of arRNA (see Table 4, each arRNA was chemically modified in the CM0 pattern) were electroporated into GM06214 cells by using the electroporation conditions in Example 2, after the electroporation, intracellular enzyme activity was respectively detected on day 2, 4, 6, 8, 10, 12 and 14, and the A to G mutation rate of the RNA target sites in the cells was also detected on day 2 and day 4 by the method in Example 3, respectively. The results are shown in Figs. 4A and 4B. The highest enzyme activity was observed when the arRNA was 91nt (45-c-45), and the IDUA enzyme activity remained at a high level on day 6 after electroporation. In terms of the A to G mutation rate, 111nt and 91nt presented approximately the same editing efficiency. While the 51nt arRNA was consistently unable to reach a desirable editing efficiency.

### Example 5: arRNA Optimization

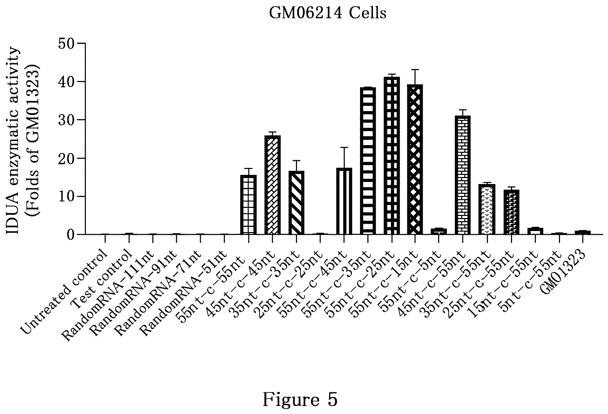

Through literature research, we believe that the electroporation approach cannot fully meet the purpose of future disease treatment. Therefore, we tested the liposome transfection method. For example, in this example, we used lipofectamineRNAiMAX (Invitrogen 13778-150) to transfect arRNA. Firstly, we designed new arRNAs by truncating the sequence at both ends on the basis of 111nt, and later the design was extended by fixing one end while truncating from the other end. By this design method, 14 arRNA sequences were generated. Four random sequences (random) were also designed as controls. The arRNA sequences used in this example are shown in Table 5 below. Each arRNA was chemically modified with the CM0 pattern. By testing the enzyme activity of IDUA at 48 h after transfection (same method as Example 3), it was found that the editing efficiency decreases when the arRNA length is equal to or more than 111 nt; and in the case of equal length of arRNA, higher editing efficiency may be achieved when the sequence lengths flanking the targeting nucleotide C are not the same, and when the length of the 5' end flanking sequence of the targeting nucleotide C is greater than that of 3' end flanking sequence, the editing efficiency tends to be higher (Fig. 5). Among them, preferably when the flanking sequence of the targeting nucleotide C at 3' end of arRNA is longer than 5 nt, particularly in the range of 5 nt-45 nt, the arRNA reaches the highest value of editing efficiency; and the flanking sequence of the targeting nucleotide C at 5' end is preferably greater than 25 nt, particularly when it exceeds 35nt, the editing efficiency of the arRNA is significantly elevated. It is worth mentioning that, the enzyme activity of arRNAs with shorter total lengths like 61nt, 51nt is consistently low.

Furthermore, in the arRNAs tested in this example, cells edited with the following arRNAs have better IDUA enzyme activity: 45nt-c-55nt (SEQ ID NO: 17) having a total length of 101nt, 55nt-c-35nt (SEQ ID NO: 13) having a total length of 91nt, 55nt-c-25nt (SEQ ID NO: 24) having a total length of 81nt, and 55nt-c-15nt (SEQ ID NO: 25) having a total length of 71 nt.

**Table 5.**

| | |
|---|---|
| 111 nt-random | SEQ ID NO: 5 uaccgcuacagccacgcugauuucagcuauaccugcccgguauaaagggacguucacaccgcg auguucucugcuggggaauugcgcgauauucaggauuaaaagaagugc |
| 91nt-random | SEQ ID NO: 6 uaauccugaauaucgcgcaauuccccagcagagaacaucgcggugugaacgucccuuuauacc |
| | gggcagguauagcugaaaucagcguggc |
| 71nt-random | SEQ ID NO: 7 uuucagcuauaccugcccgguauaaagggacguucacaccgcgauguucucugcuggggaau ugcgcgaua |
| 51nt-random | SEQ ID NO: 8 uuccccagcagagaacaucgcggugugaacgucccuuuauaccgggcaggu |
| 55nt-c-55nt | SEQ ID NO: 4 gacgcccaccgugugguugcuguccaggacggucccggccugcgacacuucggcccagagcug cuccucaucugcggggcgggggggggccgucgccgcguggggucguug |
| 45nt-c-45nt | SEQ ID NO: 9 gugguugcuguccaggacggucccggccugcgacacuucggcccagagcugcuccucauc ugcggggcgggggggggccgucgccgcgu |
| 35nt-c-35nt | SEQ ID NO: 10 uguccaggacggucccggccugcgacacuucggcccagagcugcuccucaucugcggggcggg ggggggcc |
| 25nt-c-25nt | SEQ ID NO: 11 ggucccggccugcgacacuucggcccagagcugcuccucaucugcggggcg |
| 55nt-c-45nt | SEQ ID NO: 12 gacgcccaccgugugguugcuguccaggacggucccggccugcgacacuucggcccagagcug cuccucaucugcggggcgggggggggccgucgccgcgu |
| 55nt-c-35nt | SEQ ID NO: 13 gacgcccaccgugugguugcuguccaggacggucccggccugcgacacuucggcccagagcug cuccucaucugcggggcgggggggggcc |
| 55nt-c-25nt | SEQ ID NO: 14 gacgcccaccgugugguugcuguccaggacggucccggccugcgacacuucggcccagagcug cuccucaucugcggggcg |
| 55nt-c-15nt | SEQ ID NO: 15 gacgcccaccgugugguugcuguccaggacggucccggccugcgacacuucggcccagagcug cuccucau |
| 55nt-c-5nt | SEQ ID NO: 16 gacgcccaccgugugguugcuguccaggacggucccggccugcgacacuucggcccagagc |
| 45nt-c-55nt | SEQ ID NO: 17 gugugguugcuguccaggacggucccggccugcgacacuucggcccagagcugcuccucauc ugcggggcgggggggggccgucgccgcguggggucguug |
| 35nt-c-55nt | SEQ ID NO: 18 uguccaggacggucccggccugcgacacuucggcccagagcugcuccucaucugcggggcggg ggggggccgucgccgcguggggucguug |
| 25nt-c-55nt | SEQ ID NO: 19 ggucccggccugcgacacuucggcccagagcugcuccucaucugcggggcgggggggggccg ucgccgcguggggucguug |
| 15nt-c-55nt | SEQ ID NO: 20 ugcgacacuucggcccagagcugcuccucaucugcggggcgggggggggccgucgccgcgug gggucguug |
| 5nt-c-55nt | SEQ ID NO: 21 cggcccagagcugcuccucaucugcggggcgggggggggccgucgccgcguggggucguug |

### Example 6: Screening for the Optimal Length from the Editing Site to the 3' End

The IDUA gene RNA contains a plurality of contiguous cytidines (C's) near the target site. therefore, when designing an arRNA complementary to the target RNA sequence, the counterpart to the plurality of contiguous C's is a plurality of guanosines (G's). Poly G consisting of a plurality of G's are difficult to synthesize at the current level of chemical synthesis technology, and should therefore be avoided. Since the plurality of G's are located at position 20nt from the target site (4 contiguous G's), and position 25nt (9 contiguous G's) in the direction of the 3' end of the targeting nucleotide. Therefore, in all subsequent examples, we tried to make the 3' end flanking sequence of the targeting nucleotide C of the designed arRNA less than 25nt to avoid poly G structures as much as possible, with the preferred 3' end flanking sequence of the targeting nucleotide C is less than 20nt.

Since in Example 6, we detected higher IDUA enzyme activity and A to G mutation rate for 81nt:55-c-25 and 71nt:55-c-15 sequences, reflecting their relatively high editing efficiency. To find a shorter and better length of the 3' end flanking sequence of the targeting nucleotide C, we also designed and tested sequences starting from a distance (from the editing site to the 3' end) from 25nt (81nt:55-c-25) to 10nt (66nt:55nt-c-10nt). arRNA sequences are shown in Table 6. Each arRNA was chemically modified with the CM0 pattern. GM06214 cells were transfected with lipofectamineRNAiMAX, then inoculating in 6-well plates with 3^{∗}10⁵ cells per well one day before transfection, and fresh culture medium was changed for the cells on the day of transfection. Cells were transfected with arRNA at a concentration of 20 nM. Cells were collected 48 hours after transfection for enzyme activity detection.

Enzyme activity detection method was performed as follows: GM06214 cells were digested to centrifuge, then resuspending with 33 µl of 1×PBS containing 0.1% Triton X-100. After lysis on ice for 30 min, the cells were centrifuged at 4°C for 2 min. Then 25 µl of cell lysate was added to 25 µl of substrate containing 190 µM 4-methylumbelliferyl-α-L-iduronidase (Glycosynth, 44076). The substrate was dissolved in a buffer containing 0.4 M sodium formate and 0.2% Triton X-100 (pH 3.5) to incubate at 37°C for 30 min in the dark. Afterwards, 200 µl of 0.5 M NaOH/glycine solution (pH 10.3) (Beijing Chemical Industry, NAOH, Catalog. No. AR500G; Solarbio, Glycine Catalog. No. G8200) was added to inactivate the catalytic reaction. Fluorescence values were measured by using an Infinite M200 instrument (TECAN) and excitation lights of 365 nm and 450 nm. Cell lysates were taken for concentration measurement.

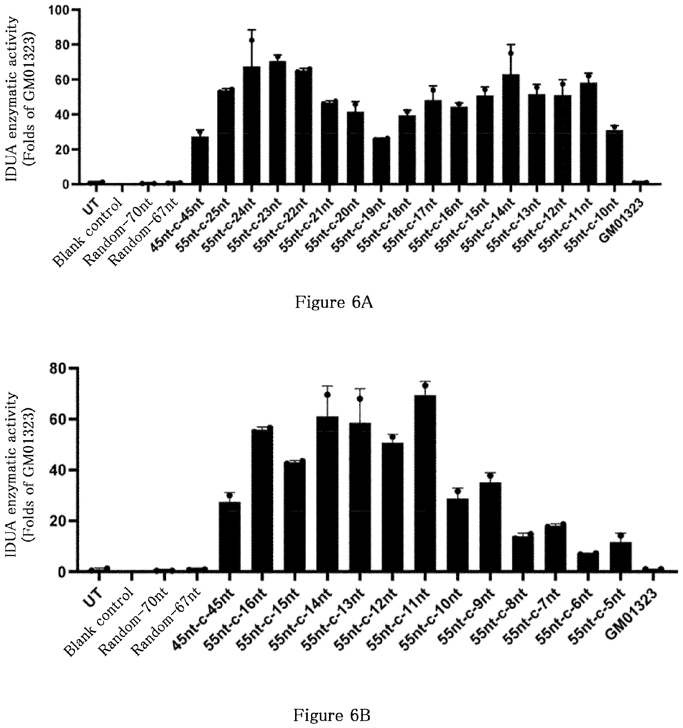

As shown in Fig. 6A, by IDUA enzyme activity measurement (results were shown as: folds of the IDUA enzyme activity in GM01323 cells), it can be seen that all arRNAs in which the distance from the targeting nucleotide to the 3' end to be 25nt to 10nt achieve more desirable editing efficiency. To further shorten the length of 3' flanking sequence of the targeting nucleotide, we synthesized a new batch of arRNAs in which the distance from the targeting nucleotide to the 3' end to be 16nt to 5nt. The results of IDUA enzyme activity are shown in Fig. 6B. The results of enzyme activity obtained after transfection of GM06214 cells with the same arRNA sequences for 48 h in the 2 experimental results were basically the same. In addition, the arRNAs with 7nt to10nt length of 3' end flanking sequence of the targeting nucleotide also show relatively good editing efficiency, and the arRNAs with 9nt-10nt length of 3' end flanking sequence of the targeting nucleotide are more effective. It can also be seen from the results in Fig. 6B that, the editing efficiency of arRNAs with total length below 61nt is always lower than expected.

Combining the data of Example 6 with the results of Figs. 6A and 6B, it can be seen that the length of 3' end flanking sequence of the targeting nucleotide of an arRNA should preferably be limited in the range of 9nt-25nt. And the total length of the arRNA should not be lower than 61nt.

**Table 6.**

| | |
|---|---|
| 55nt-c-25nt | SEQ ID NO: 14: gacgcccaccgugugguugcuguccaggacggucccggccugcgacacuucggcccagagcugcucc ucaucugcggggcg |
| 55nt-c-24nt | SEQ ID NO: 22: gacgcccaccgugugguugcuguccaggacggucccggccugcgacacuucggcccagagcugcucc ucaucugcggggc |
| 55nt-c-23nt | SEQ ID NO: 23: gacgcccaccgugugguugcuguccaggacggucccggccugcgacacuucggcccagagcugcucc ucaucugcgggg |
| 55nt-c-22nt | SEQ ID NO: 24: gacgcccaccgugugguugcuguccaggacggucccggccugcgacacuucggcccagagcugcucc ucaucugcggg |
| 55nt-c-21nt | SEQ ID NO: 25: gacgcccaccgugugguugcuguccaggacggucccggccugcgacacuucggcccagagcugcucc ucaucugcgg |
| 55nt-c-20nt | SEQ ID NO: 26: gacgcccaccgugugguugcuguccaggacggucccggccugcgacacuucggcccagagcugcucc ucaucugcg |
| 55nt-c-19nt | SEQ ID NO: 27: gacgcccaccgugugguugcuguccaggacggucccggccugcgacacuucggcccagagcugcucc ucaucugc |
| 55nt-c-18nt | SEQ ID NO: 28: gacgcccaccgugugguugcuguccaggacggucccggccugcgacacuucggcccagagcugcucc ucaucug |
| 55nt-c-17nt | SEQ ID NO: 29gacgcccaccgugugguugcuguccaggacggucccggccugcgacacuucggcccagagcugc uccucaucu |
| 55nt-c-16nt | SEQ ID NO: 30: gacgcccaccgugugguugcuguccaggacggucccggccugcgacacuucggcccagagcugcucc ucauc |
| 55nt-c-15nt | SEQ ID NO: 15: gacgcccaccgugugguugcuguccaggacggucccggccugcgacacuucggcccagagcugcucc ucau |
| 55nt-c-14nt | SEQ ID NO: 31 gacgcccaccgugugguugcuguccaggacggucccggccugcgacacuucggcccagagcugcucc uca |
| 55nt-c-13nt | SEQ ID NO: 32: gacgcccaccgugugguugcuguccaggacggucccggccugcgacacuucggcccagagcugcucc |
| | uc |
| 55nt-c-12nt | SEQ ID NO: 33: gacgcccaccgugugguugcuguccaggacggucccggccugcgacacuucggcccagagcugcucc u |
| 55nt-c-11nt | SEQ ID NO: 34: gacgcccaccgugugguugcuguccaggacggucccggccugcgacacuucggcccagagcugcucc |
| 55nt-c-10nt | SEQ ID NO: 35: gacgcccaccgugugguugcuguccaggacggucccggccugcgacacuucggcccagagcugcuc |
| 55nt-c-9nt | SEQ ID NO: 36: gacgcccaccgugugguugcuguccaggacggucccggccugcgacacuucggcccagagcugcu |
| 55nt-c-8nt | SEQ ID NO: 37: gacgcccaccgugugguugcuguccaggacggucccggccugcgacacuucggcccagagcugc |
| 55nt-c-7nt | SEQ ID NO: 38: gacgcccaccgugugguugcuguccaggacggucccggccugcgacacuucggcccagagcug |
| 55nt-c-6nt | SEQ ID NO: 39: gacgcccaccgugugguugcuguccaggacggucccggccugcgacacuucggcccagagcu |
| 55nt-c-5nt | SEQ ID NO: 16: gacgcccaccgugugguugcuguccaggacggucccggccugcgacacuucggcccagagc |
| random-70nt | SEQ ID NO: 40: uaccgcuacagccacgcugauuucagcuauaccugcccgguauaaagggacguucacaccgcgaugu ucu |
| random-67nt | SEQ ID NO: 41: uaccgcuacagccacgcugauuucagcuauaccugcccgguauaaagggacguucacaccgcgaug |

### Example 7: Screening for Optimal 5' End Length under the Condition that the Preferred 3' End Length was Fixed

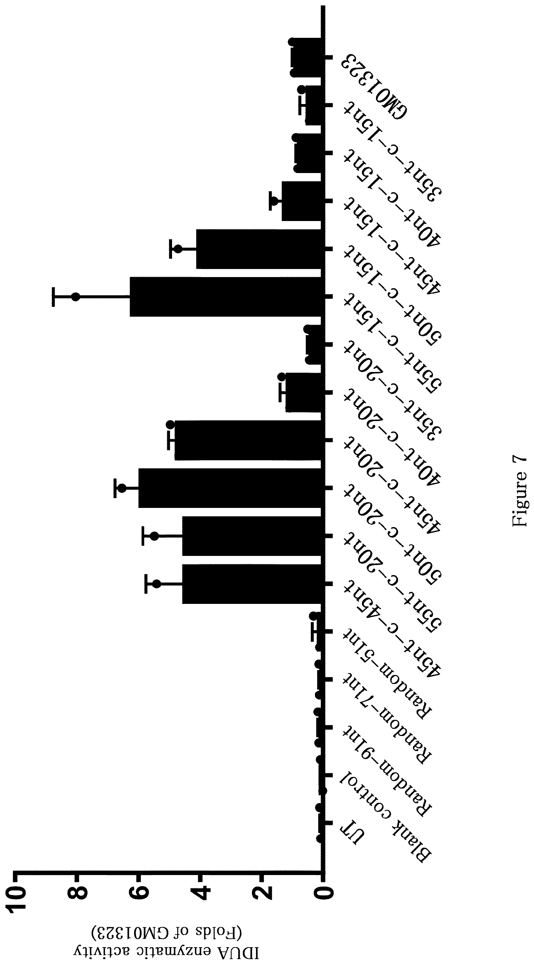

We selected 2 different lengths of arRNAs: 76nt:55-c-20, 71nt:55-c-15, with a fixed length of the 3' end flanking sequence of the targeting nucleotide, the 5' end was gradually truncated, as shown in Table 7. Each arRNA was chemically modified with the CM0 pattern. After transfecting GM06214 cells with lipofectamine RNAiMAX, the IDUA enzyme activity was measured with tests. The results are shown in Fig. 7. When the total length of arRNA was lower than 61nt, its editing efficiency was dramatically reduced. While in the case that total length of the arRNA is greater than 61nt, the IDUA enzyme activity of cells after editing with the arRNA is significantly increased when the length of 3' end flanking sequence of the targeting nucleotide is 15nt or 20nt, and the length of 5' end flanking sequence of the targeting nucleotide is greater than 40nt. As shown in Fig. 7.

**Table 7.**

| | |
|---|---|
| 55nt-c-20nt | SEQ ID NO: 26: |
| | gacgcccaccgugugguugcuguccaggacggucccggccugcgacacuucggcccagagcugcuccucauc ugcg |
| 50nt-c-20nt | SEQ ID NO: 42: ccaccgugugguugcuguccaggacggucccggccugcgacacuucggcccagagcugcuccucaucugcg |
| 45nt-c-20nt | SEQ ID NO: 43: gugugguugcuguccaggacggucccggccugcgacacuucggcccagagcugcuccucaucugcg |
| 40nt-c-20nt | SEQ ID NO: 44: guugcuguccaggacggucccggccugcgacacuucggcccagagcugcuccucaucugcg |
| 35nt-c-20nt | SEQ ID NO: 45: uguccaggacggucccggccugcgacacuucggcccagagcugcuccucaucugcg |
| 55nt-c-15nt | SEQ ID NO: 15: gacgcccaccgugugguugcuguccaggacggucccggccugcgacacuucggcccagagcugcuccucau |
| 50nt-c-15nt | SEQ ID NO: 46: ccaccgugugguugcuguccaggacggucccggccugcgacacuucggcccagagcugcuccucau |
| 45nt-c-15nt | SEQ ID NO: 47: gugugguugcuguccaggacggucccggccugcgacacuucggcccagagcugcuccucau |
| 40nt-c-15nt | SEQ ID NO: 48: guugcuguccaggacggucccggccugcgacacuucggcccagagcugcuccucau |
| 35nt-c-15nt | SEQ ID NO: 49: uguccaggacggucccggccugcgacacuucggcccagagcugcuccucau |

### Example 8: Study of the Effect of Targeting Nucleotide Position Shift on the Editing Efficiency of the Target Site

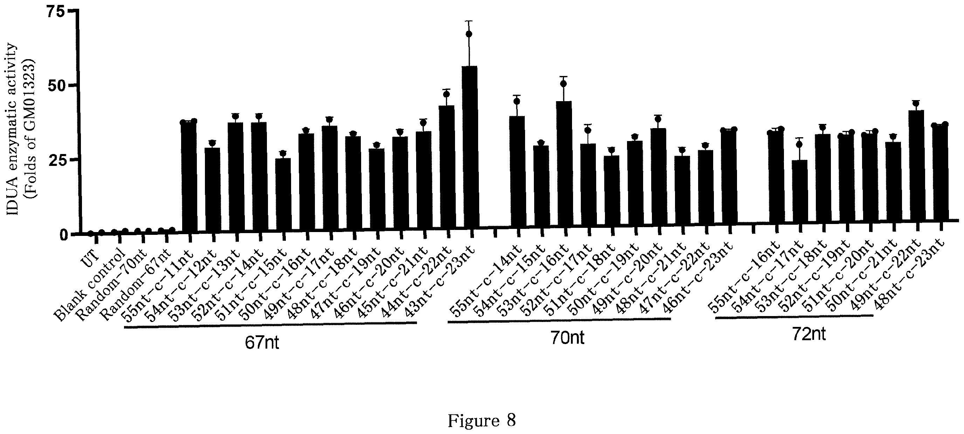

Three lengths of arRNAs were selected from the previous examples to study the effect of targeting nucleotide position shift on the editing efficiency of the target site. A series of arRNA sequences were designed by gradually moving the targeting nucleotide from the central position to the 3' end direction, while considering the requirement of arRNA synthesis to avoid some unfavorable structures for synthesis. arRNAs are shown in Table 8. Each arRNA was chemically modified with the CM0 pattern. Samples were collected for enzyme activity detection (the enzyme activity detection was performed according to the method in Example 7, and the results were shown as folds of the IDUA enzyme activity in GM01323) 48hrs after transfecting GM06214 cells with lipofectamineRNAiMAX. The experimental results are shown in Fig. 8. When moving the targeting nucleotide position on arRNAs with the total lengths of 67nt, 70nt and 72nt, the movement of the targeting nucleotide position did not have a significant effect on the enzyme activity, the total length was kept constant, and the length of the 5' end flanking sequence of the targeting nucleotide was 43nt-55nt, and the length of the 3' end flanking sequence of the targeting nucleotide was 9nt-25nt. That is, this Example demonstrates that a relatively excellent editing efficiency was achieved for each arRNA within the aforementioned preferred range of arRNA lengths, 5' lengths, and 3' lengths.

**Table 8.**

| | | |
|---|---|---|
| 67nt | 55nt-c-11nt | gacgcccaccgugugguugcuguccaggacggucccggccugcgacacuucggccca gagcugcucc (SEQ ID NO: 34) |
| | 54nt-c- 12nt | acgcccaccgugugguugcuguccaggacggucccggccugcgacacuucggcccag agcugcuccu (SEQ ID NO: 50) |
| | 53nt-c-13nt | cgcccaccgugugguugcuguccaggacggucccggccugcgacacuucggcccaga gcugcuccuc (SEQ ID NO: 51) |
| | 52nt-c-14nt | gcccaccgugugguugcuguccaggacggucccggccugcgacacuucggcccagag cugcuccuca (SEQ ID NO: 52) |
| | 51nt-c-15nt | cccaccgugugguugcuguccaggacggucccggccugcgacacuucggcccagagc ugcuccucau (SEQ ID NO: 53) |
| | 50nt-c-16nt | ccaccgugugguugcuguccaggacggucccggccugcgacacuucggcccagagcu gcuccucauc (SEQ ID NO: 54) |
| | 49nt-c-17nt | caccgugugguugcuguccaggacggucccggccugcgacacuucggcccagagcug cuccucaucu (SEQ ID NO: 55) |
| | 48nt-c-18nt | accgugugguugcuguccaggacggucccggccugcgacacuucggcccagagcugc uccucaucug (SEQ ID NO: 56) |
| | 47nt-c-19nt | ccguguggguugcuguccaggacggucccggccugcgacacuucggcccagagcugcu ccucaucugc (SEQ ID NO: 57) |
| | 46nt-c-20nt | cgugugguugcuguccaggacggucccggccugcgacacuucggcccagagcugcuc cucaucugcg (SEQ ID NO: 58) |
| | 45nt-c-21nt | gugugguugcuguccaggacggucccggccugcgacacuucggcccagagcugcucc ucaucugcgg (SEQ ID NO: 59) |
| | 44nt-c-22nt | ugugguugcuguccaggacggucccggccugcgacacuucggcccagagcugcuccu caucugcggg (SEQ ID NO: 60) |
| | 43nt-c-23nt | gugguugcuguccaggacggucccggccugcgacacuucggcccagagcugcuccuc aucugcgggg (SEQ ID NO: 61) |
| 70nt | 55nt-c-14nt | gacgcccaccgugugguugcuguccaggacggucccggccugcgacacuucggccca gagcugcuccuca (SEQ ID NO: 31) |
| | 54nt-c-15nt | acgcccaccgugugguugcuguccaggacggucccggccugcgacacuucggcccag agcugcuccucau (SEQ ID NO: 62) |
| | 53nt-c-16nt | cgcccaccgugugguugcuguccaggacggucccggccugcgacacuucggcccaga gcugcuccucauc (SEQ ID NO: 63) |
| | 52nt-c-17nt | gcccaccgugugguugcuguccaggacggucccggccugcgacacuucggcccagag cugcuccucaucu (SEQ ID NO: 64) |
| | 51nt-c-18nt | cccaccgugugguugcuguccaggacggucccggccugcgacacuucggcccagagc ugcuccucaucug (SEQ ID NO: 65) |
| | 50nt-c-19nt | ccaccgugugguugcuguccaggacggucccggccugcgacacuucggcccagagcu gcuccucaucugc (SEQ ID NO: 66) |
| | 49nt-c-20nt | caccgugugguugcuguccaggacggucccggccugcgacacuucggcccagagcug |
| | | cuccucaucugcg (SEQ ID NO: 67) |
| | 48nt-c-21nt | accgugugguugcuguccaggacggucccggccugcgacacuucggcccagagcugc uccucaucugcgg (SEQ ID NO: 68) |
| | 47nt-c-22nt | ccgugugguugcuguccaggacggucccggccugcgacacuucggcccagagcugcu ccucaucugcggg (SEQ ID NO: 69) |
| | 46nt-c-23nt | cgugugguugcuguccaggacggucccggccugcgacacuucggcccagagcugcuc cucaucugcgggg (SEQ ID NO: 70) |
| 72nt | 55nt-c-16nt | gacgcccaccgugugguugcuguccaggacggucccggccugcgacacuucggccca gagcugcuccucauc (SEQ ID NO: 30) |
| | 54nt-c-17nt | acgcccaccgugugguugcuguccaggacggucccggccugcgacacuucggcccag agcugcuccucaucu (SEQ ID NO: 71) |
| | 53nt-c-18nt | cgcccaccgugugguugcuguccaggacggucccggccugcgacacuucggcccaga gcugcuccucaucug (SEQ ID NO: 72) |
| | 52nt-c- 19nt | gcccaccgugugguugcuguccaggacggucccggccugcgacacuucggcccagag cugcuccucaucugc (SEQ ID NO: 73) |
| | 51nt-c-20nt | cccaccgugugguugcuguccaggacggucccggccugcgacacuucggcccagagc ugcuccucaucugcg (SEQ ID NO: 74) |
| | 50nt-c-21nt | ccaccgugugguugcuguccaggacggucccggccugcgacacuucggcccagagcu gcuccucaucugcgg (SEQ ID NO: 75) |
| | 49nt-c-22nt | caccgugugguugcuguccaggacggucccggccugcgacacuucggcccagagcug cuccucaucugcggg (SEQ ID NO: 76) |
| | 48nt-c-23nt | accgugugguugcuguccaggacggucccggccugcgacacuucggcccagagcugc uccucaucugcgggg (SEQ ID NO: 77) |

### Example 9: Effect of arRNA Chemical Modifications on Editing Efficiency

Firstly, we compared the effect of CM1 modification versus CM0 modification on arRNA editing efficiency in three lengths of arRNAs (55-c-16 (SEQ ID NO: 30), 55-c-14 (SEQ ID NO: 31), and 55-c-11 (SEQ ID NO: 34)). The CM1 modifications are shown below:
CM1: the first 3 and the last 3 nucleotides in the arRNA sequence were respectively modified by 2'-O-methylation, the first 3 and the last 3 internucleotide linkages in the arRNA sequence were respectively modified by phosphorothioation, and all the uridines of the sequence were modified by 2'-O-methylation.

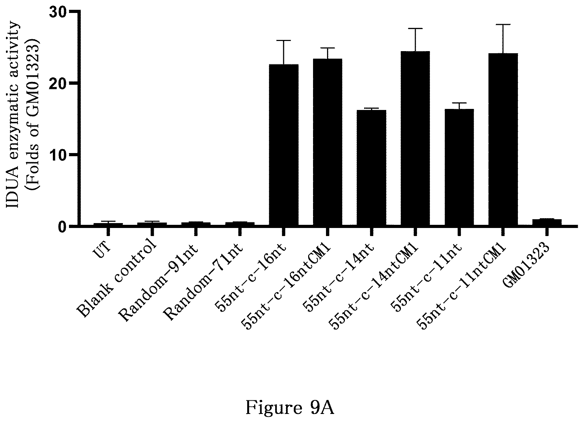

The results are shown in Fig. 9A, and the editing efficiency of CM1-modified arRNAs is generally better than that of CM0-modified arRNAs.

Subsequently, we then selected 2 preferred lengths of arRNAs: 71nt, and 76nt, and investigated the effect of different combinations of modifications on the editing efficiency. The specific modifications are shown below, and the specific sequences and modification annotations are shown in Table 9.

CM2: the first 3 and the last 3 nucleotides in the arRNA sequence were respectively modified by 2'-O-methylation, and the first 3 and last 3 internucleotide linkages in the arRNA sequence were respectively modified by phosphorothioation; and the 3' most adjacent nucleotide of the targeting nucleotide was modified by 2'-O-methylation.

CM3: the first 3 and the last 3 nucleotides in the arRNA sequence were respectively modified by 2'-O-methylation, the first 3 and the last 3 internucleotide linkages in the arRNA sequence were respectively modified by phosphorothioation, and the 5' most adjacent nucleotide of the targeting nucleotide was modified by 2'-O-methylation.

CM4: the first 3 and the last 3 nucleotides in the arRNA sequence were respectively modified by 2'-O-methylation, the first 3 and the last 3 internucleotide linkages in the arRNA sequence were respectively modified by phosphorothioation, and the 3' most adjacent internucleotide linkage of each of the three bases in the targeting triplet was modified by phosphorothioation.

CM5: All nucleotides were modified by 2'-OMe except the targeting nucleoside and the 5 bases adjacent to its 5' end and the 5 bases adjacent to its 3' end; and the first and last three internucleotide linkages of the sequence were modified by phosphorothioation.

CM6: the first 5 and the last 5 nucleotides in the arRNA sequence were respectively modified by 2'-O-methylation, and the first 5 and the last 5 internucleotide linkages of the sequence were respectively modified by phosphorothioation.

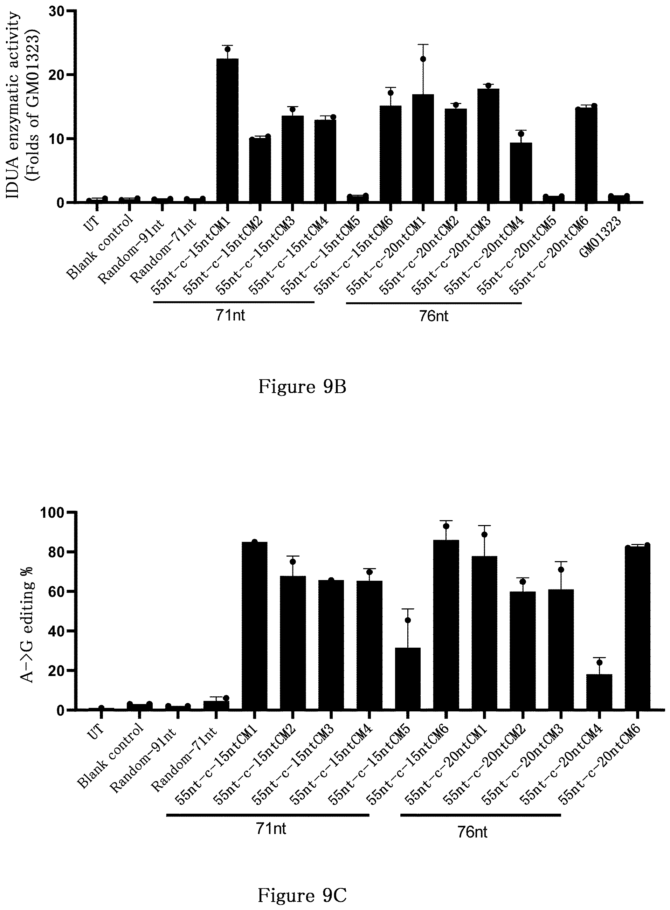

IDUA mRNA was edited by transfecting different arRNAs into GM06214 cells separately using Lipofectamine RNAiMAX. 48 h after transfection, cells were collected for IDUA enzyme activity detection (enzyme activity detection was performed according to the method in Example 7, and results were shown as folds of the enzyme activity in GM01323). It can be seen from the experimental results shown in Fig. 9B, except for CM5, other combinations of modifications show good editing efficiency as compared to CM1 modification.

The IDUA enzyme activity detection allows comparison of the ability of different lengths of arRNA to restore IDUA protein function at the protein level. In order to be able to quickly probe the editing ability of arRNA at the RNA level, the editing efficiency of arRNA was also detected in this example by using an enzymatic cleavage method. The specific method is as follows: the normal sequence of the site near the IDUA mRNA point mutation contains a CTGG, which becomes CTAG after mutation. Particularly, the four bases CTAG are the restriction sites of the restriction endonuclease BfaI, so if the transfected arRNA implements the A to G editing on the target site in the cell, the restriction site CTAG of the restriction endonuclease BfaI will be transformed into CTGG, and thus the enzymatic cleavage of BfaI will no longer occur. 48 hours after cell transfection with arRNA, the cell genome was extracted to perform reverse transcription. Samples were subjected to PCR by using primers hIDUA-62F: CCTTCCTGAGCTACCACCCG (SEQ ID NO: 78), and hIDUA-62R: CCAGGGCTCGAACTCGGTAG (SEQ ID NO: 79). PCR products were purified and recovered for enzymatic cleavage. The enzymatically cleaved products were subjected to gel electrophoresis with 2% agarose, and the editing efficiency was calculated by calculating the percentage of the gray value of uncut fragments relative to the overall gray value on the gel map. As shown in Fig. 9C, the editing efficiency results show a trend consistent with the enzyme activity results of the arRNAs.

**Table 9.**

| Name | Length | Modification | Sequence |
|---|---|---|---|
| HIV2-76-CM1 | 55nt-c-20nt | CM1 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C-C- A-G-A-G-C-Um-G-C-Um-C-C-Um-C-A-Um-C-Um*Gm *Cm*Gm (SEQ ID NO: 26) |
| HIV2-76-CM2 | 55nt-c-20nt | CM2 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C-C- Am-G-A-G-C-Um-G-C-Um-C-C-Um-C-A-Um-C-Um*G m*Cm*Gm (SEQ ID NO: 26) |
| HIV2-76-CM3 | 55nt-c-20nt | CM3 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-Cm- C-A-G-A-G-C-Um-G-C-Um-C-C-Um-C-A-Um-C-Um* Gm*Cm*Gm (SEQ ID NO: 26) |
| HIV2-76-CM4 | 55nt-c-20nt | CM4 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C*C* A*G-A-G-C-Um-G-C-Um-C-C-Um-C-A-Um-C-Um*Gm *Cm*Gm (SEQ ID NO: 26) |
| HIV2-76-CM5 | 55nt-c-20nt | CM5 | Gm*Am*Cm*Gm-Cm-Cm-Cm-Am-Cm-Cm-Gm-Um-G m-Um-Gm-Gm-Um-Um-Gm-Cm-Um-Gm-Um-Cm-Cm- Am-Gm-Gm-Am-Cm-Gm-Gm-Um-Cm-Cm-Cm-Gm-Gm -Cm-Cm-Um-Gm-Cm-Gm-Am-Cm-Am-Cm-Um-Um-C- |
| | | | G-G-C-C-C-A-G-A-G-C-Um-Gm-Cm-Um-Cm-Cm-Um- Cm-Am-Um-Cm-Um*Gm*Cm*Gm (SEQ ID NO: 26) |
| HIV2-76-CM6 | 55nt-c-20nt | CM6 | Gm*Am*Cm*Gm*Cm*C-C-A-C-C-G-U-G-U-G-G-U-U -G-C-U-G-U-C-C-A-G-G-A-C-G-G-U-C-C-C-G-G-C-C- U-G-C-G-A-C-A-C-U-U-C-G-G-C-C-C-A-G-A-G-C-U- G-C-U-C-C-U-C-A-U*Cm*Um*Gm*Cm*Gm (SEQ ID NO: 26) |
| HIV2-71-CM1 | 55nt-c-15nt | CM1 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C-C- A-G-A-G-C-Um-G-C-Um-C-C-Um*Cm*Am*Um (SEQ ID NO: 15) |
| HIV2-71-CM2 | 55nt-c-15nt | CM2 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C-C- Am-G-A-G-C-Um-G-C-Um-C-C-Um*Cm*Am*Um (SEQ ID NO: 15) |
| HIV2-71-CM3 | 55nt-c-15nt | CM3 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-Cm- C-A-G-A-G-C-Um-G-C-Um-C-C-Um*Cm*Am*Um (SEQ ID NO: 15) |
| HIV2-71-CM4 | 55nt-c-15nt | CM4 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C*C* A*G-A-G-C-Um-G-C-Um-C-C-Um*Cm*Am*Um (SEQ ID NO: 15) |
| HIV2-71-CM5 | 55nt-c-15nt | CM5 | Gm*Am*Cm*Gm-Cm-Cm-Cm-Am-Cm-Cm-Gm-Um-G m-Um-Gm-Gm-Um-Um-Gm-Cm-Um-Gm-Um-Cm-Cm- Am-Gm-Gm-Am-Cm-Gm-Gm-Um-Cm-Cm-Cm-Gm-Gm -Cm-Cm-Um-Gm-Cm-Gm-Am-Cm-Am-Cm-Um-Um-C- G-G-C-C-C-A-G-A-G-C-Um-Gm-Cm-Um-Cm-Cm-Um* Cm*Am*Um (SEQ ID NO: 15) |
| HIV2-71-CM6 | 55nt-c-15nt | CM6 | Gm*Am*Cm*Gm*Cm*C-C-A-C-C-G-U-G-U-G-G-U-U -G-C-U-G-U-C-C-A-G-G-A-C-G-G-U-C-C-C-G-G-C-C- U-G-C-G-A-C-A-C-U-U-C-G-G-C-C-C-A-G-A-G-C-U- G-C-U-C*Cm*Um*Cm*Am*Um (SEQ ID NO: 15) |

| | | | |
|---|---|---|---|
| Note: "m" indicates 2'-O-Me on nucleotide ribose, and "*" indicates phosphorothioation modification. | | | |

### Example 10: Concentration Gradient Tests on CM1-modified 55nt-c-16nt, 55nt-c-14nt, and 55nt-c-11nt

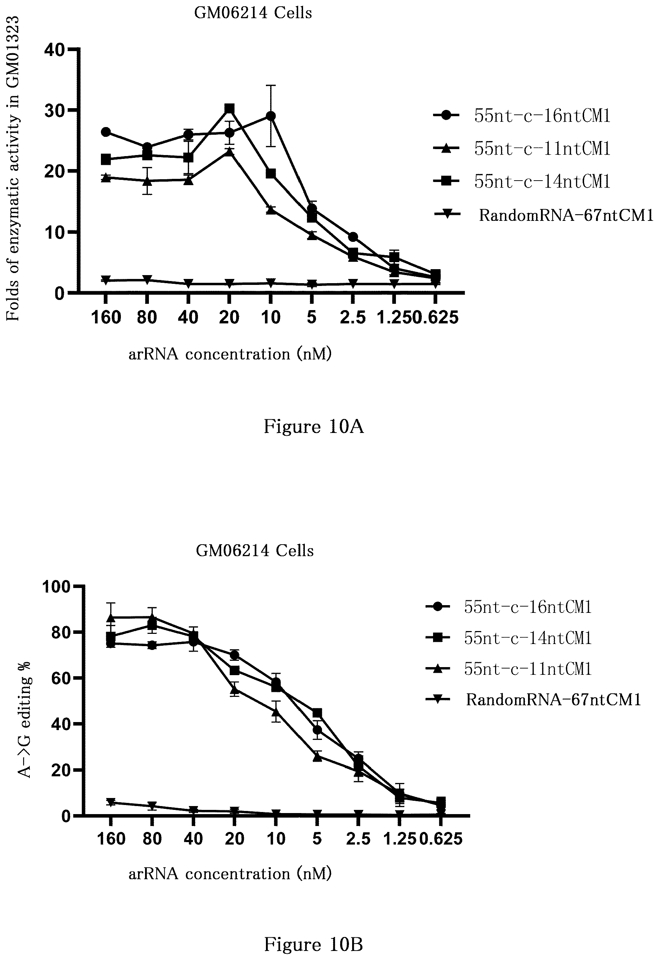

To investigate the editing ability of arRNA at different concentrations, CM1-modified 55nt-c-16nt, 55nt-c-14nt, and 55nt-c-11nt were selected for testing in this example. A total of 9 different dilutions of arRNA at 160nM, 80nM, 40nM, 20nM, 10nM, 5nM, 2.5nM, 1.25nM, and 0.625nM were taken, and GM06214 cells were transfected by using lipofectamine RNAiMAX. Forty-eight hours after transfection, the cells were detected for enzyme activity, and gene editing efficiency was detected by second-generation sequencing. As shown in Fig. 10A, the overall trend of the three arRNAs is basically the same in terms of enzyme activity results. 55nt-c-16nt-CM1 has higher enzyme activity than the other two arRNAs at transfection concentrations greater than 10 nM. However, the inflection point concentrations for enzyme activity are all between 20 nM and 5 nM. Below 5nM, the editing efficiency is no longer satisfactory. The result trend of editing efficiency detected by second-generation sequencing tends to be consistent with the enzyme activity overall, as shown in Fig. 10B. It can be seen that the concentration of arRNA transfected by using liposomes is preferably greater than or equal to 5nM, preferably greater than or equal to 10nM, and more preferably greater than or equal to 20nM.

### Example 11: IDUA Sustains Protease Activity after Editing by arRNA

In this experiment, by using three preferred arRNAs targeting human IDUA target sites, and chemically modifying them with the CM1 pattern, significantly increased IDUA enzyme activity was obtained in GM06214 cells and lasted for more than about 3 weeks.

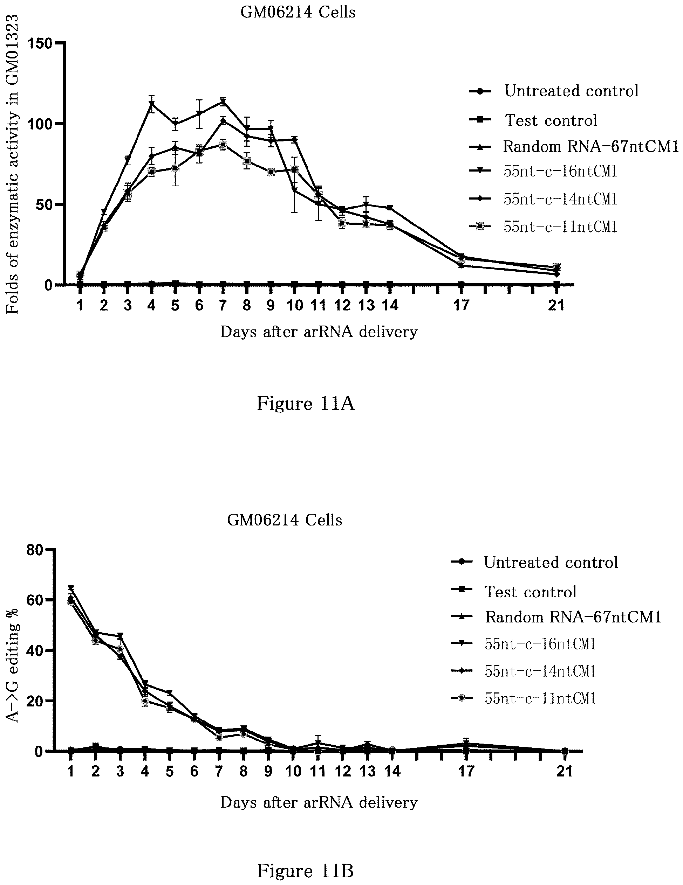

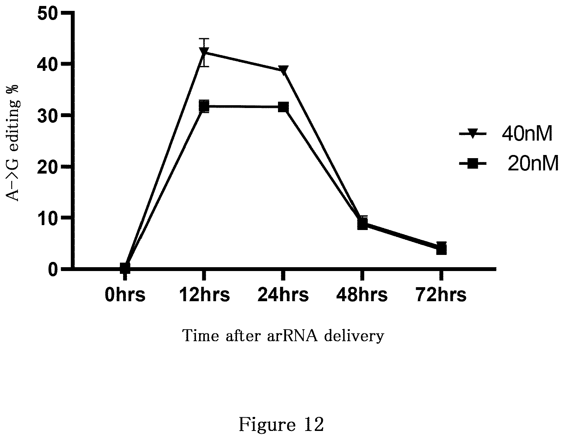

We selected a concentration of 20 nM, and the IDUA enzyme activity and editing efficiency at different times were compared. As shown in Fig. 11A, after transfecting the arRNAs to GM06214 cells, IDUA enzyme activity was detected consecutively for 14 days. The results show that the peak of enzyme activity after transfection appears from day 4 to day 10, and mainly appears from day 4 to day 9. And the enzyme activity on day 14 is still higher than that on day 2, and then we examined the enzyme activity on days 17 and 21, and it can be seen from Fig. 12A that the enzyme activity on day 21 is still higher than that on day 1 after transfection, and the enzyme activity is about 6 to 10 times of that ofGM01323. The editing efficiency of IDUA target sites is shown in Fig. 11B, from which it can be seen that from high to low the editing efficiency after arRNA transfection was about 70% of editing as the highest peak on day 1 after transfection, untill day 10 after transfection the editing efficiency is equal to that of the control group.

Therefore, it is easy to speculate that if a second transfection is performed on days 10 to 14, it will be possible to consistently maintain the IDUA enzyme activity at a higher level than that on the second day of the first transfection. And if the second transfection is performed at days 17 to 21, it would allow the IDUA enzyme activity to be at least higher than the enzyme activity in GM01323, keeping the IDUA enzyme activity of the cells in a relatively normal state.

### Example 12: In Vitro Editing of the Wild IDUA (c.1205-5bp) Site with arRNA in Human Primary Hepatocytes

This test involves editing of the wild-type IDUA gene site in cultured human primary hepatocytes by LEAPER technology. In this example, A in the CAG was used as a target for editing to test the editing efficiency of the method according to this application for IDUA gene in hepatocytes. The arRNA sequences are shown in Table 10.

Human primary hepatocytes were purchased from LONZA (Catalog. No. HUCPI), and the cells were recovered and cultured according to the manufacturer's instructions (recovery medium Catalog. No. MCHT50; plating medium Catalog. No. MP100). The cells were replaced with hepatocyte maintaining medium 5C after adherence (reference document: Xiang C, Du Y, Meng G, et al. Long-term functional maintenance of primary human hepatocytes in vitro[J]. Science, 2019, 364(6438):399-402).

After 24h of adherent culture, human hepatocytes were transfected with arRNAs by using lipofectmine RNAiMAX, wherein the transfection concentrations of arRNAs were 20 nM and 40 nM, respectively. Samples were respectively collected at 0 h, 12 h, 24 h, 48 h and 72 h after transfection. After collection, samples were assayed for editing efficiency by second-generation sequencing. As shown in Fig. 12, after the transfection of arRNA, the editing efficiency of IDUA can reach up to 40%. It demonstrates the feasibility and effectiveness of editing the target adenosine of IDUA transcripts in human hepatocytes by the method according to this application.

**Table 10.**

| arRNA name | arRNA length | modific ation | sequence |
|---|---|---|---|
| hIV2-wt1 CM1 | 55nt-c-14nt | CM1 | Cm*Am*Cm*-C-G-Um-G-Um-G-G-Um-Um-G-C-Um-G-Um-C- C-A-G-G-A-C-G-G-Um-C-C-C-G-G-C-C-Um-G-C-G-A-C-A-C- Um-Um-C-G-G-C-C-C-A-G-A-G-C-C-G-C-Um-C-C-Um-C-A-U |
| | | | m-C-Um*Gm*Cm*Gm (SEQ ID NO: 80) |

### Example 13: Comparison of Combinations of Chemical Modification Methods and Lengths

In this Example, by editing the c.1239 G>A (p.Trp402Ter) mutation site in GM06214 cells, we compared the method in this application with the preferred method in the prior art. The advantages of the method according to this application were further shown.

The sequences and modifications are shown in Table 11. The representative sequence of the method according to this application is 55nt-c-15nt-CM1, and the positive control sequence selected from the prior art (CN110352244A) is 36nt-c-13nt-CM11, wherein all the nucleotides, except the editing site CCA, were modified by 2'-O-Me, and the first and last 4 nucleotide linkages were modified by phosphorothioation. In addition, the CMC-modified sequence (55nt-c-15nt) and the CM0-modified random sequence (Random-70) in this application were taken as control in this example.

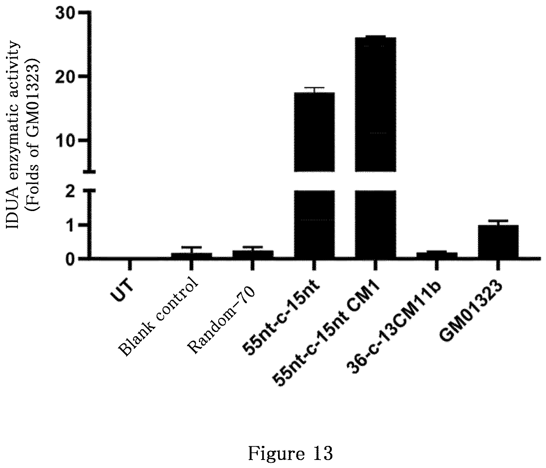

In this example, 48 hours after transfection of arRNA into GM06214 cells by using LipofectamineRNAiMAX, the IDUA enzyme activity was detected by the method shown in Example 7. The results are shown in Fig. 13. It can be seen that, 55nt-c-15nt-CM1 has a significantly higher editing efficiency than that of 36nt-c-13nt-CM11.

**Table 11.**

| Name | Modification | Sequence |
|---|---|---|
| 55nt-c-15 nt-CM1 | CM1 | Gm*Am*Cm*G-C-C-C-A-C-C-G-Um-G-Um-G-G-Um-Um- G-C-Um-G-Um-C-C-A-G-G-A-C-G-G-Um-C-C-G-G-C-C -Um-G-C-G-A-C-A-C-Um-Um-C-G-G-C-C-A-G-A-G-C- Um-G-C-Um-C-C-Um*Cm*Am*Um (SEQ ID NO:15) |
| 36nt-c-13 nt-CM 11 | CM11 | Cm*Um*Gm*Um*Cm-Cm-Am-Gm-Gm-Am-Cm-Gm-Gm- Um-Cm-Cm-Cm-Gm-Gm-Cm-Cm-Um-Gm-Cm-Gm-Am-C m-Am-Cm-Um-Um-Cm-Gm-Gm-Cm-C-C-A-Gm-Am-Gm-C m-Um-Gm-Cm-Um*Cm*Cm*Um*Cm (SEQ ID NO:81) |

| | | |
|---|---|---|
| Note: "m" indicates 2'-O-Me modification on nucleotide ribose, and "*" refers to phosphorothioation modification. | | |

### Example 14: Effect of End Modifications on arRNA Editing Efficiency

In this Example, different amounts of 2'MOE and LNA modifications were performed on both ends of arRNAs based on CM1, and they were compared with CM1 modifications, wherein 2'MOE refers to 2'-O-(2-methoxyethyl) modification.

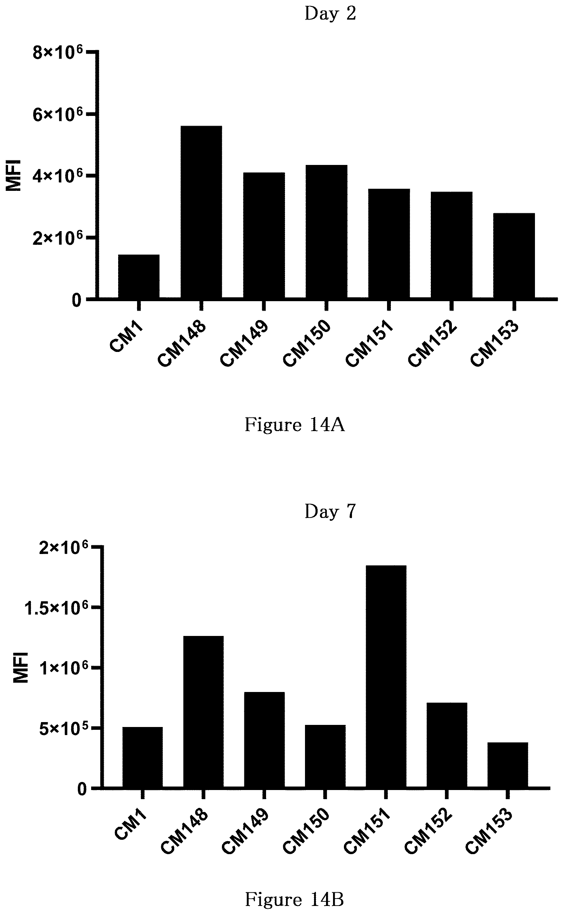

As shown in Table 12, the sequence 55nt-c-20nt (SEQ ID NO: 26) was modified with CM1, CM148, CM149, CM150, CM151, CM152 and CM153, respectively, for testing in this Example. LipofectamineRNAiMAX was used in this example to transfect arRNA into IDUA reporter cells at a transfection concentration of 20 nM. Then the fluorescence intensity was measured by flow cytometry after 48h (day 2) and 168h (day 7), respectively. The results are shown in Fig. 14. It can be seen that CM151 has significantly higher editing efficiency, followed by CM148. In addition, CM148, CM149, CM150, CM151, CM152, and CM153 are all superior to the CM1 modification method.

The specific modification methods used in this example (except CM1 which is previously described) are shown below:
CM148: the first 4 and the last 4 nucleotides in the arRNA sequence were respectively modified by 2'-O-(2-methoxyethyl), the first 3 and the last 4 internucleotide linkages in the arRNA sequence were respectively modified by phosphorothioation, and all the uridines of the sequence were modified by 2'-O-Me.
CM149: the first 3 and the last 3 nucleotides in the arRNA sequence were respectively modified by 2'-O-(2-methoxyethyl), the first 2 and the last 3 internucleotide linkages in the arRNA sequence were respectively modified by phosphorothioation, and all the uridines of the sequence were modified by 2'-O-Me.
CM150: the first 2 and the last 2 nucleotides in the arRNA sequence were respectively modified by 2'-O-(2-methoxyethyl), the first and the last 2 internucleotide linkages in the arRNA sequence were respectively modified by phosphorothioation, and all the uridines of the sequence were modified by 2'-O-Me.
CM151: the first 4 and the last 4 nucleotides in the arRNA sequence were respectively modified by LNA, the first 3 and the last 4 internucleotide linkages in the arRNA sequence were respectively modified by phosphorothioation, and all the uridines of the sequence were modified by 2'-O-Me.
CM152: the first 3 and the last 3 nucleotides in the arRNA sequence were respectively modified by LNA, the first 2 and the last 3 internucleotide linkages in the arRNA sequence were respectively modified by phosphorothioation, and all the uridines of the sequence were modified by 2'-O-Me.
CM153: the first 2 and the last 2 nucleotides in the arRNA sequence were respectively modified by LNA, the first 2 and the last 3 internucleotide linkages in the arRNA sequence were respectively modified by phosphorothioation, and all the uridines of the sequence were modified by 2'-O-Me.

**Table 12.**

| Name | Length | Modification | Sequence |
|---|---|---|---|
| HIV2-7 0-CM1 | 55nt-c-14nt | CM1 | Gm*Am*Cm*G-C-C-C-A-C-C-G-Um-G-Um-G-G-Um-Um- G-C-Um-G-Um-C-C-A-G-G-A-C-G-G-Um-C-C-C-G-G-C-C- Um-GC-G-A-C-A-C-Um-Um-C-G-G-C-C-C-A-G-A-G-C-Um -G-C-Um-C-C*Um*Cm*Am (SEQ ID NO: 31) |
| HIV2-7 0-CM1 48 | 55nt-c-14nt | CM148 | Gmoe*Amoe*Cmoe*Gmoe-C-C-C-A-C-C-G-Um-G-Um-G-G -Um-Um-G-C-Um-G-Um-C-C-A-G-G-A-C-G-G-Um-C-C-C- G-G-C-C-Um-GC-G-A-C-A-C-Um-Um-C-G-G-C-C-C-A-G- A-G-C-Um-G-C-Um-C*Cmoe*Umoe*Cmoe*Amoe (SEQ ID NO: 31) |
| HIV2-7 0-CM1 49 | 55nt-c-14nt | CM149 | Gmoe*Amoe*Cmoe-G-C-C-C-A-C-C-G-Um-G-Um-G-G-Um -Um-G-C-Um-G-Um-C-C-A-G-G-A-C-G-G-Um-C-C-C-G-G- C-C-Um-GC-G-A-C-A-C-Um-Um-C-G-G-C-C-C-A-G-A-G- C-Um-G-C-Um-CC*Umoe*Cmoe*Amoe (SEQ ID NO: 31) |
| HIV2-7 0-CM1 50 | 55nt-c-14nt | CM150 | Gmoe*Amoe-C-G-C-C-C-A-C-C-G-Um-G-Um-G-G-Um-Um -G-C-Um-G-Um-C-C-A-G-G-A-C-G-G-Um-C-C-C-G-G-C-C -Um-GC-G-A-C-A-C-Um-Um-C-G-G-C-C-A-G-A-G-C-U m-G-C-Um-CCU*Cmoe*Amoe (SEQ ID NO: 31) |
| HIV2-7 0-CM1 51 | 55nt-c-14nt | CM151 | G+*A+*C+*G+C-C-C-A-C-C-G-Um-G-Um-G-G-Um-Um-G -C-Um-G-Um-C-C-A-G-G-A-C-G-G-Um-C-C-C-G-G-C-C-U m-GC-G-A-C-A-C-Um-Um-C-G-G-C-C-C-A-G-A-G-C-Um- G-C-Um-C*C+*U+*C+*A+ (SEQ ID NO: 31) |
| HIV2-7 0-CM1 52 | 55nt-c-14nt | CM152 | G+*A+*C+G-C-C-C-A-C-C-G-Um-G-Um-G-G-Um-Um-G-C -Um-G-Um-C-C-A-G-G-A-C-G-G-Um-C-C-C-G-G-C-C-Um- GC-G-A-C-A-C-Um-Um-C-G-G-C-C-A-G-A-G-C-Um-G- C-Um-C-C*U+*C+*A+ (SEQ ID NO: 31) |
| HIV2-7 0-CM1 53 | 55nt-c-14nt | CM153 | G+*A+*C-G-C-C-C-A-C-C-G-Um-G-Um-G-G-Um-Um-G-C -Um-G-Um-C-C-A-G-G-A-C-G-G-Um-C-C-C-G-G-C-C-Um- GC-G-A-C-A-C-Um-Um-C-G-G-C-C-A-G-A-G-C-Um-G- C-Um-C-C*U*C+*A+ (SEQ ID NO: 31) |

| | | | |
|---|---|---|---|
| Note: "m" indicates 2'-O-Me modification on nucleotide ribose; "*" indicates phosphorothioation modification; "+" indicates locking nucleic acid (LNA) modification. "Moe" indicates 2'-O-(2-methoxyethyl) modification. | | | |

### Example 15: Effect of Modifications with Combinations of Phosphorothioation, 2'-O-(2-methoxyethyl), and Deoxyribonucleotide Substitution on arRNA Editing Efficiency

This example attempts to add more phosphorothioation and 2'-O-Me modifications to arRNA, and introduces deoxyribonucleotide substitution.

The specific sequence modifications are as follows:
CM71: the first 3 and last 3 nucleotides in the arRNA sequence were each modified by 2'-OMe, while in the arRNA sequence, all the uridines and all the cytidines except that in the targeting triplet were modified by 2'-OMe, and the first 3 and last 3 internucleotide linkages were modified by phosphorothioation.
CM94: the first 3 and the last 3 nucleotides in the arRNA sequence, all the uridines and all the cytidines except that in the targeting triplet in the arRNA sequence were modified by 2'-O-methylation; the first 4 and the last 3 internucleotide linkages in the arRNA sequence were all modified by phosphorothioation; and from the 4th internucleotide linkage from the 5' end to the 3rd internucleotide linkage from the 3' end, every other internucleotide linkage was modified by phosphorothioation, except that the 5' most adjacent internucleotide linkage of each nucleotide in the targeting triple was not modified by phosphorothioation.
CM104: the first 3 and last 3 nucleotides in the arRNA sequence were each modified by 2'-O-methylation, while in the arRNA sequence, all the uridines and all the cytidines except that in the targeting triplet were modified by 2'-O-methylation, and all the nucleotide linkages were modified by phosphorothioation except that the 5' most adjacent internucleotide linkage of each nucleotide in the targeting triple was not modified by phosphorothioation.
CM105: in addition to comprising all the modifications in CM104, the targeting nucleotide C was modified by deoxyribonucleotide substitution.
CM106: in addition to comprising all the modifications in CM104, each nucleotide in the targeting triplet was modified by deoxyribonucleotide substitution.
CM107: in addition to comprising all the modifications in CM94, the targeting nucleotide C was modified by deoxyribonucleotide substitution.
CM108: in addition to comprising all the modifications in CM94, each nucleotide in the targeting triplet was modified by deoxyribonucleotide substitution.
CM114: in addition to comprising all the modifications in CM94, the 5' most adjacent internucleotide linkage of each nucleotide in the targeting triple was also modified by phosphorothioation.
CM115: in addition to comprising all the modifications in CM94, the 3' most adjacent internucleotide linkage of each nucleotide in the targeting triple was also modified by phosphorothioation.
CM116: in addition to comprising all the modifications in CM94, the 5' most adjacent internucleotide linkage of the 5' most adjacent nucleotide of the targeting nucleotide was also modified by phosphorothioation.
CM117: in addition to comprising all the modifications in CM94, the 3' most adjacent internucleotide linkage of the targeting nucleotide was also modified by phosphorothioation.
CM118: in addition to comprising all the modifications in CM115, the targeting nucleotide C was modified by deoxyribonucleotide substitution.
CM119: except that the 5' most adjacent internucleotide linkage of the targeting nucleotide C was not modified by phosphorothioation, other modifications were the same as those in CM118.
CM120: in addition to comprising all the modifications in CM116, the targeting nucleotide C was modified by deoxyribonucleotide substitution.
CM121: in addition to comprising all the modifications in CM117, the targeting nucleotide C was modified by deoxyribonucleotide substitution.
CM122: in addition to comprising all the modifications in CM114, each nucleotide in the targeting triplet was modified by deoxyribonucleotide substitution.
CM123: each nucleotides in the targeting triplet was modified by deoxyribonucleotide substitution, while other modifications were the same as those in CM119.
CM124: in addition to comprising all the modifications in CM116, each nucleotide in the targeting triplet was modified by deoxyribonucleotide substitution.
CM125: in addition to comprising all the modifications in CM117, each nucleotide in the targeting triplet was modified by deoxyribonucleotide substitution.

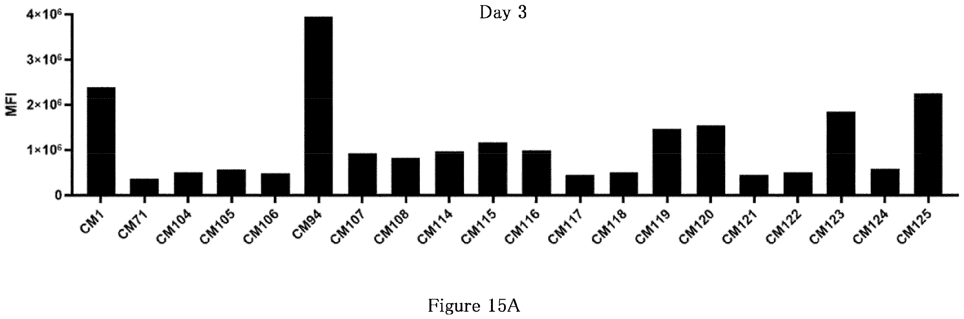

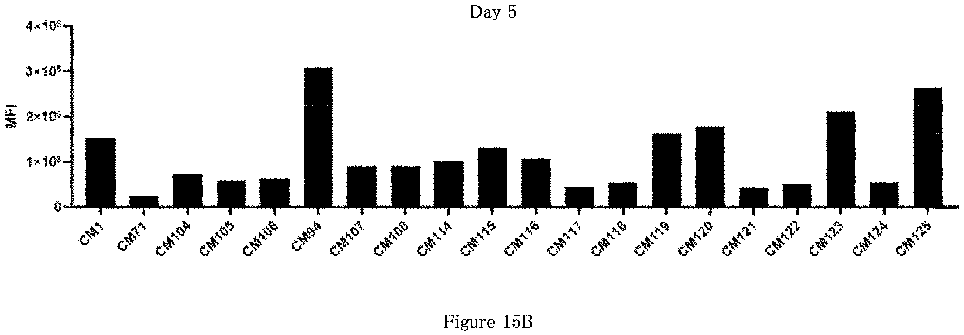

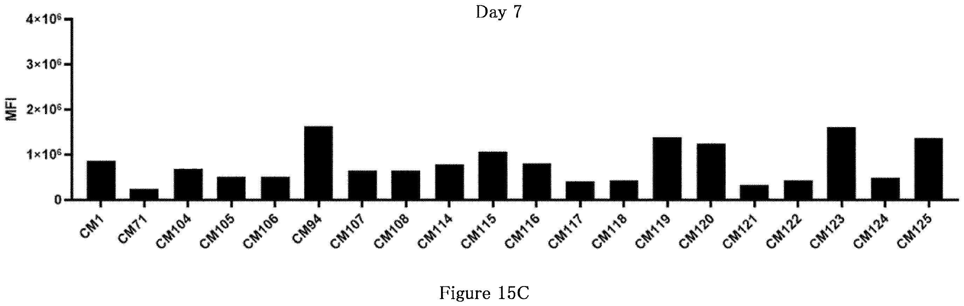

The sequence 55nt-c-20nt (SEQ ID NO: 26) and modification annotations used in this example are shown in Table 13. LipofectamineRNAiMAX was used in this example to transfect arRNA into IDUA Reporter cells at a transfection concentration of 20 nM. The fluorescence intensity was detected by flow cytometry after 72, 120 and 168 hours, respectively. The results are shown in Fig. 15, CM94, CM123, and CM125 has significantly higher editing efficiency, in addition, CM119 and CM120 are more stable relative to CM1 and they maintain higher editing efficiency for a longer period of time.

**Table 13**

| Name | Length | Modification | Sequence |
|---|---|---|---|
| HIV2-70 -CM1 | 55nt-c-14nt | CM1 | Gm*Am*Cm*G-C-C-C-A-C-C-G-Um-G-Um-G-G-Um- Um-G-C-Um-G-Um-C-C-A-G-G-A-C-G-G-Um-C-C-C- G-G-C-C-Um-G-C-G-A-C-A-C-Um-Um-C-G-G-C-C-C- A-G-A-G-C-Um-G-C-Um-C-C*Um*Cm*Am (SEQ ID NO: 31) |
| HIV2-70 -CM71 | 55nt-c-14nt | CM71 | Gm*Am*Cm*G-Cm-Cm-Cm-A-Cm-Cm-G-Um-G-Um- G-G-Um-Um-G-Cm-Um-G-Um-Cm-Cm-A-G-G-A-Cm- G-G-Um-Cm-Cm-Cm-G-G-Cm-Cm-Um-G-Cm-G-A-Cm -A-Cm-Um-Um-Cm-G-G-Cm-C-C-A-G-A-G-Cm-Um-G -Cm-Um-Cm-Cm*Um*Cm*Am (SEQ ID NO: 31) |
| HIV2-70 -CM94 | 55nt-c-14nt | CM94 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm-C-C-A*G-A*G -Cm*Um-G*Cm-Um*Cm-Cm*Um*Cm*Am (SEQ ID NO: 31) |
| HIV2-70 -CM104 | 55nt-c-14nt | CM104 | Gm*Am*Cm*G*Cm*Cm*Cm*A*Cm*Cm*G*Um*G* Um*G*G*Um*Um*G*Cm*Um*G*Um*Cm*Cm*A*G* G*A*Cm*G*G*Um*Cm*Cm*Cm*G*G*Cm*Cm*Um* G*Cm*G*A*Cm*A*Cm*Um*Um*Cm*G*G*Cm-C-C- A*G*A*G*Cm*Um*G*Cm*Um*Cm*Cm*Um*Cm*A m (SEQ ID NO: 31) |
| HIV2-70 -CM105 | 55nt-c-14nt | CM105 | Gm*Am*Cm*G*Cm*Cm*Cm*A*Cm*Cm*G*Um*G* Um*G*G*Um*Um*G*Cm*Um*G*Um*Cm*Cm*A*G* G*A*Cm*G*G*Um*Cm*Cm*Cm*G*G*Cm*Cm*Um* G*Cm*G*A*Cm*A*Cm*Um*Um*Cm*G*G*Cm-C-Cd -A*G*A*G*Cm*Um*G*Cm*Um*Cm*Cm*Um*Cm*A m (SEQ ID NO: 31) |
| HIV2-70 -CM106 | 55nt-c-14nt | CM106 | Gm*Am*Cm*G*Cm*Cm*Cm*A*Cm*Cm*G*Um*G* Um*G*G*Um*Um*G*Cm*Um*G*Um*Cm*Cm*A*G* G*A*Cm*G*G*Um*Cm*Cm*Cm*G*G*Cm*Cm*Um* G*Cm*G*A*Cm*A*Cm*Um*Um*Cm*G*G*Cm-Cd-C d-Ad*G*A*G*Cm*Um*G*Cm*Um*Cm*Cm*Um*Cm* Am (SEQ ID NO: 31) |
| HIV2-70 -CM107 | 55nt-c-14nt | CM107 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm-C-Cd-A*G-A* |
| | | | G-Cm*Um-G*Cm-Um*Cm-Cm*Um*Cm*Am (SEQ ID NO: 31) |
| HIV2-70 -CM108 | 55nt-c-14nt | CM108 | Gm*Am*Cm*G*Cm-Cm*Cm-A*Cm-Cm*G-Um*G-Um *G-G*Um-Um*G-Cm*Um-G*-Um-Cm*Cm-A*G-G*-A -Cm*G-G*Um-Cm*Cm-Cm*G-G*Cm-Cm*Um-G*Cm- G*A-Cm*A-Cm*Um-Um*Cm-G*G-Cm-Cd-Cd-Ad*G- A*-G-Cm*Um-G*Cm-Um*Cm-Cm*Um*Cm*Am (SEQ ID NO: 31) |
| HIV2-70 -CM114 | 55nt-c-214nt | CM114 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm*C*C*A*G-A* G-Cm*Um-G*Cm-Um*Cm-Cm*Um*Cm*Am (SEQ ID NO: 31) |
| HIV2-70 -CM115 | 55nt-c-14nt | CM115 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm-C*C*A*G-A* G-Cm*Um-G*Cm-Um*Cm-Cm*Um*Cm*Am (SEQ ID NO: 31) |
| HIV2-70 -CM116 | 55nt-c-14nt | CM116 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm*C-C-A*G-A* G-Cm*Um-G*Cm-Um*Cm-Cm*Um*Cm*Am (SEQ ID NO: 31) |
| HIV2-70 -CM117 | 55nt-c-14nt | CM117 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm-C-C*A*G-A* G-Cm*Um-G*Cm-Um*Cm-Cm*Um*Cm*Am (SEQ ID NO: 31) |
| HIV2-70 -CM118 | 55nt-c-14nt | CM118 | Gm*Am*Cm*G*Cm-Cm*Cm-A*-Cm-Cm*G-Um*G-U m*G-G*Um-Um*G-Cm*Um-G*Um-Cm*Cm-A*G-G*A -Cm*G-G*Um-Cm*Cm-Cm*G-G*Cm-Cm*Um-G*Cm- G*A-Cm*A-Cm*Um-Um*Cm-G*G-Cm*C*Cd*A*G-A *G-Cm*Um-G*Cm-Um*Cm-Cm*Um*Cm*Am     (SEQ ID NO: 31) |
| HIV2-70 -CM119 | 55nt-c-14nt | CM119 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm*C-Cd*A*G-A* G-Cm*Um-G*Cm-Um*Cm-Cm*Um*Cm*Am (SEQ ID NO: 31) |
| HIV2-70 -CM120 | 55nt-c-14nt | CM120 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm*C-Cd-A*G-A* G-Cm*Um-G*Cm-Um*Cm-Cm*Um*Cm*Am (SEQ ID NO: 31) |
| HIV2-70 -CM121 | 55nt-c-14nt | CM121 | Gm*Am*Cm*G*-Cm-Cm*Cm-A*Cm-Cm*G-Um*G-U m*G-G*-Um-Um*G-Cm*Um-G*Um-Cm*Cm-A*G-G* A-Cm*-G-G*Um-Cm*Cm-Cm*G-G*Cm-Cm*Um-G*C m-G*A-Cm*A-Cm*Um-Um*Cm-G*G-Cm-C-Cd*A*-G -A*-G-Cm*Um-G*Cm-Um*Cm-Cm*Um*Cm*Am (SEQ ID NO: 31) |
| HIV2-70 -CM122 | 55nt-c-14nt | CM122 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*G-G*Um-Cm*Cm-Cm*G-G*Cm-Cm*Um-G*Cm- G*A-Cm*A-Cm*Um-Um*Cm-G*G-Cm*Cd*Cd*Ad*G- A*G-Cm*Um-G*Cm-Um*Cm-Cm*Um*Cm*Am (SEQ ID NO: 31) |
| HIV2-70 -CM123 | 55nt-c-14nt | CM123 | Gm*Am*Cm*G*Cm-Cm*Cm-A*-Cm-Cm*G-Um*G-U m*G-G*Um-Um*G-Cm*Um-G*Um-Cm*Cm-A*G-G*A -Cm*G-G*Um-Cm*Cm-Cm*G-G*Cm-Cm*Um-G*Cm- G*-A-Cm*A-Cm*Um-Um*Cm-G*G-Cm*Cd-Cd*Ad*- G-A*G-Cm*Um-G*Cm-Um*Cm-Cm*Um*Cm*Am (SEQ ID NO: 31) |
| HIV2-70 -CM124 | 55nt-c-14nt | CM124 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*-G-G*Um-Cm*Cm-Cm*G-G*Cm-Cm*Um-G*Cm- G*A-Cm*A-Cm*Um-Um*Cm-G*G-Cm*Cd-Cd-Ad*G- A*G-Cm*Um-G*Cm-Um*Cm-Cm*Um*Cm*Am (SEQ ID NO: 31) |
| HIV2-70 -CM125 | 55nt-c-14nt | CM125 | Gm*Am*Cm*G*Cm-Cm*Cm-A*Cm-Cm*G-Um*G-Um *G-G*Um-Um*G-Cm*Um-G*Um-Cm*Cm-A*G-G*A- Cm*-G-G*Um-Cm*Cm-Cm*G-G*Cm-Cm*Um-G*-Cm- G*A-Cm*A-Cm*Um-Um*Cm-G*G-Cm-Cd-Cd*Ad*G- A*G-Cm*Um-G*Cm-Um*Cm-Cm*Um*Cm*Am (SEQ ID NO: 31) |

| | | | |
|---|---|---|---|
| "m" indicates 2'-O-methylation modification on nucleotide ribose; "*" indicates phosphorothioation modification; "d" indicates deoxyribonucleotide substitution modification. | | | |

## Claims

1. A method for targeted editing of a target RNA in a cell based on the LEAPER technology, wherein the target RNA comprises a transcript sequence of an α-L-iduronidase (IDUA) gene containing a guanosine (G) to adenosine (A) mutation site (target adenosine), and the method comprises:
introducing into the cell an arRNA or a construct comprising the arRNA coding sequence, wherein the arRNA comprises a complementary RNA sequence that hybridizes to the target RNA, and wherein the arRNA is capable of recruiting an adenosine deaminase acting on RNA (ADAR) by hybridization to the target RNA so as to deaminate the target adenosine in the target RNA.

2. The method according to claim 1, wherein the nucleotide (targeting nucleotide) in the arRNA opposite to the target adenosine is a cytidine (C), an adenosine (A) or a uridine (U).

3. The method according to claim 1 or 2, wherein the target adenosine forms a target triplet together with its 5' most adjacent nucleotide and 3' most adjacent nucleotide, and the target triplet is 5'-UAG-3'.

4. The method according to any one of claims 1-3, wherein the 5' most adjacent nucleotide of the targeting nucleotide is a cytidine (C), a guanosine (G) or a uridine (U).

5. The method according to any one of claims 1-4, wherein the 3' most adjacent nucleotide of the targeting nucleotide is an adenosine (A).

6. The method according to any one of claims 1-5, wherein the targeting nucleotide forms a targeting triplet together with its 5' most adjacent nucleotide and 3' most adjacent nucleotide, and the targeting triplet is 5'-CCA-3'.

7. The method according to any one of claims 1-6, wherein the length of the arRNA is any integer number of nt selected from 61nt to 121nt.

8. The method according to any one of claims 1-7, wherein the distance from the targeting nucleotide of the arRNA to the 3' end of the arRNA is less than the distance from the targeting nucleotide to the 5' end of the arRNA.

9. The method according to any one of claims 1-8, wherein the distance from the targeting nucleotide of the arRNA to the 3' end of the arRNA is any integer number of nt selected from 9nt to 60nt.

10. The method according to any one of claims 1-9, wherein the distance from the targeting nucleotide of the arRNA to the 5' end of the arRNA is any integer number of nt selected from 25nt to 60nt.

11. The method according to any one of claims 1-10, wherein the arRNA is an arRNA having a length characteristic selected from the group consisting of:
55nt-c-35nt, 55nt-c-25nt, 55nt-c-24nt, 55nt-c-21nt, 55nt-c-20nt, 55nt-c-19nt, 55nt-c-18nt, 55nt-c-17nt, 55nt-c-16nt, 55nt-c-15nt, 55nt-c-14nt, 55nt-c-13nt, 55nt-c-12nt, 55nt-c-11nt, 55nt-c-10nt, 55nt-c-9nt, 50nt-c-20nt, 50nt-c-15nt, and 45nt-c-20nt;
wherein cytidine is the targeting nucleotide in the arRNA opposite to the target adenosine, and the orientation of the arRNA is 5' to 3'.

12. The method according to any one of claims 1-11, wherein the arRNA is an arRNA having a length charactorastic selected from the group consisting of: 55nt-c-20nt, 55nt-c-15nt, and 55nt-c-14nt.

13. The method according to any one of claims 1-12, wherein the arRNA comprises one or more chemical modifications.

14. The method according to claim 13, wherein the chemical modification is one or more selected from the group consisting of:
2'-O-methylation modification, phosphorothioation modification in internucleotide linkage, deoxyribonucleotide substitution modification, LNA modification, and 2'-O-(2-methoxyethyl) modification.

15. The method according to claim 13 or 14, wherein the chemical modification is one or more selected from the group consisting of:
1) 2'-O-methylation modifications in the first 2, 3, 4 or 5 nucleotides;
2) 2'-O-methylation modifications in the last 2, 3, 4, or 5 nucleotides;
3) 2'-O-methylation modifications in all the cytidines except that in the targeting triplet;
4) 2'-O-(2-methoxyethyl) modifications in the first 2, 3 or 4 nucleotides;
5) 2'-O-(2-methoxyethyl) modifications in the last 2, 3 or 4 nucleotides;
6) deoxyribonucleotide substitution modifications of the first 2, 3 or 4 nucleotides;
7) deoxyribonucleotide substitution modifications of the last 2, 3 or 4 nucleotides;
8) phosphorothioation modifications in the first 1, 2, 3, 4 or 5 internucleotide linkages;
9) phosphorothioation modifications in the last 1, 2, 3, 4 or 5 internucleotide linkages;
10) phosphorothioation modifications in all internucleotide linkages except that one or more of the 5' or 3' most adjacent internucleotide linkages of each of the nucleotides in the targeting triplet are not modified by phosphorothioation; and
11) phosphorothioation modifications in internucleotide linkages at an interval of 1, 2, 3, or 4 nucleotides except that one or more of the 5' or 3' most adjacent internucleotide linkages of each of the nucleotides in the targeting triplet are not modified by phosphorothioation.

16. The method according to any one of claims 13-15, wherein the arRNA comprises any chemical modification selected from the group consisting of:
CMC: the first 3 and the last 3 nucleotides in the arRNA sequence are respectively modified by 2'-O-methylation, and the first 3 and the last 3 internucleotide linkages in the arRNA sequence are respectively modified by phosphorothioation;
CM1: the first 3 and the last 3 nucleotides in the arRNA sequence are respectively modified by 2'-O-methylation, the first 3 and the last 3 internucleotide linkages in the arRNA sequence are respectively modified by phosphorothioation, and all the uridines of the sequence are modified by 2'-O-methylation;
CM2: the first 3 and the last 3 nucleotides in the arRNA sequence are respectively modified by 2'-O-methylation, and the first 3 and last 3 internucleotide linkages in the arRNA sequence are respectively modified by phosphorothioation; and the 3' most adjacent nucleotide of the targeting nucleotide is modified by 2'-O-methylation;
CM3: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 5' most adjacent nucleotide of the targeting nucleotide is modified by 2'-O-methylation;
CM4: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 3' most adjacent internucleotide linkage of each of the three bases in the targeting triplet is respectively modified by phosphorothioation;
CM6: the first 5 and the last 5 nucleotides in the arRNA sequence are respectively modified by 2'-O-methylation, and the first 5 and the last 5 internucleotide linkages in the arRNA sequence are respectively modified by phosphorothioation;
CM148: the first 4 and the last 4 nucleotides in the arRNA sequence are respectively modified by 2'-O-(2-methoxyethyl), the first 3 and the last 4 internucleotide linkages in the arRNA sequence are respectively modified by phosphorothioation, and all the uridines of the sequence are modified by 2'-O-Me;
CM149: the first 3 and the last 3 nucleotides in the arRNA sequence are respectively modified by 2'-O-(2-methoxyethyl), the first 2 and the last 3 internucleotide linkages in the arRNA sequence are respectively modified by phosphorothioation, and all the uridines of the sequence are modified by 2'-O-Me;
CM150: the first 2 and the last 2 nucleotides in the arRNA sequence are respectively modified by 2'-O-(2-methoxyethyl), the first 1 and the last 2 internucleotide linkages in the arRNA sequence are respectively modified by phosphorothioation, and all the uridines of the sequence are modified by 2'-O-Me;
CM151: the first 4 and the last 4 nucleotides in the arRNA sequence are respectively modified by LNA, the first 3 and the last 4 internucleotide linkages in the arRNA sequence are respectively modified by phosphorothioation, and all the uridines of the sequence are modified by 2'-O-Me;
CM152: the first 3 and the last 3 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
CM153: the first 2 and the last 2 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
CM94: the first 3 and the last 3 nucleotides in the arRNA sequence, all the uridines and all the cytidines except that in the targeting triplet in the arRNA sequence are modified by 2'-O-methylation; the first 4 and the last 3 internucleotide linkages in the arRNA sequence are all modified by phosphorothioation; and from the 4th internucleotide linkage from the 5' end to the 3rd internucleotide linkage from the 3' end, every other internucleotide linkage is modified by phosphorothioation, except that the 5' most adjacent internucleotide linkage of each nucleotide in the targeting triple is not modified by phosphorothioation;
CM119: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages in the arRNA sequence are all modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and the targeting nucleotide is substituted by a deoxyribonucleotide;
CM120: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages in the arRNA sequence are modified by phosphorothioation; from the 4th internucleotide linkage from the 5' end to the 5' most adjacent internucleotide linkage of the targeting nucleotide, and from the 3rd internucleotide linkage from the 3' end to the 3' most adjacent internucleotide linkage of the targeting nucleotide, every other internucleotide linkage is modified by phosphorothioation; and the targeting nucleotide is substituted by a deoxyribonucleotide;
CM123: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages are modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide;
CM125: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages and the 3' most adjacent nucleotide linkages of the target nucleotide are modified by phosphorothioation; from the 4th internucleotide linkage from the 5' end to the 5' most adjacent nucleotide of the targeting nucleotide's 5' most adjacent nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide.

17. The method according to any one of claims 1-16, wherein the guanosine (G) to adenosine (A) mutation site targeted by the arRNA is a mutation site in NM_000203.4(IDUA)-c.1205G>A (p.Trp402Ter), or a corresponding mutation site thereof, in the genome of a patient with Hurler syndrome.

18. The method according to any one of claims 1-17, wherein the arRNA comprises any sequence selected from the group consisting of: 
1)Gm*Am*Cm-G*Cm-Cm*Cm-A*Cm-Cm*G-Um*G-Um*G-G*Um-Um*G-Cm*Um-G*Um-
Cm*Cm-A*G-G*A-Cm*G-G*Um-Cm*Cm-Cm*G-G*Cm-Cm*Um-G*Cm-G*A-Cm*A-Cm*Um-U
m*Cm-G*G-Cm-C-C-A*G-A*G-Cm*Um-G*Cm-Um*Cm-Cm*Um*Cm*Am (SEQ ID NO: 31);
 
2)Gm*Am*Cm-G*Cm-Cm*Cm-A*-Cm-Cm*G-Um*G-Um*G-G*Um-Um*G-Cm*Um-G*Um
-Cm*Cm-A*G-G*A-Cm*G-G*Um-Cm*Cm-Cm*G-G*Cm-Cm*Um-G*Cm-G*-A-Cm*A-Cm*Um-
Um*Cm-G*G-Cm*Cd-Cd*Ad*-G-A*G-Cm*Um-G*Cm-Um*Cm-Cm*Um*Cm*Am (SEQ ID NO:
31);
 3)Gm*Am*Cm-G*Cm-Cm*Cm-A*Cm-Cm*G-Um*G-Um*G-G*Um-Um*G-Cm*Um-G*Um- 
Cm*Cm-A*G-G*A-Cm*-G-G*Um-Cm*Cm-Cm*G-G*Cm-Cm*Um-G*-Cm-G*A-Cm*A-Cm*Um-
Um*Cm-G*G-Cm-Cd-Cd*Ad*G-A*G-Cm*Um-G*Cm-Um*Cm-Cm*Um*Cm*Am (SEQ ID NO:
31);
 
4)Gmoe*Amoe*Cmoe*Gmoe-C-C-C-A-C-C-G-Um-G-Um-G-G-Um-Um-G-C-Um-G-Um-C-C
-A-G-G-A-C-G-G-Um-C-C-C-G-G-C-C-Um-GC-G-A-C-A-C-Um-Um-C-G-G-C-C-C-A-G-A-G-C-
Um-G-C-Um-C*Cmoe*Umoe*Cmoe*Amoe (SEQ ID NO: 31); or
 
5)G+*A+*C+*G+C-C-C-A-C-C-G-Um-G-Um-G-G-Um-Um-G-C-Um-G-Um-C-C-A-G-G-A-
C-G-G-Um-C-C-C-G-G-C-C-Um-GC-G-A-C-A-C-Um-Um-C-G-G-C-C-C-A-G-A-G-C-Um-G-C-U
m-C*C+*U+*C+*A+ (SEQ ID NO: 31);
 wherein * indicates a phosphorothioation modification, m indicates a 2'-O-methylation modification, moe indicates a 2'-O-(2-methoxyethyl) modification, + indicates an LNA modification, and d indicates a deoxyribonucleotide substitution modification.

19. The method according to any one of claims 1-18, wherein the arRNA is introduced into the cells by any method selected from the group consisting of: electroporation, liposome transfection, exosome delivery, or lipid-nanoparticle delivery (LNP).

20. The method according to any one of claims 1-19, wherein the amount of arRNA in a single introduction into the cells is 5 to 160 nM.

21. The method according to any one of claims 1-20, wherein several introductions are taken for introducing the arRNA into the cells, and the interval between the adjacent two introductions is ≤ 21 days.

22. A method for preventing or treating Hurler syndrome, comprising correcting a pathogenic G>A mutation of Hurler syndrome by using the method according to any one of claims 1-21.

23. An engineered arRNA which is used for targeted editing of a target RNA in a cell based on LEAPER technology, wherein the target RNA comprises a transcript sequence of an α-L-iduronidase (IDUA) gene containing a guanosine (G) to adenosine (A) mutation site (target adenosine), the arRNA comprises a complementary RNA sequence that hybridizes to the target RNA, and the arRNA is capable of recruiting an adenosine deaminase acting on RNA (ADAR) by hybridization to the target RNA so as to deaminate the target adenosine in the target RNA, the guanosine (G) to adenosine (A) mutation site targeted by the arRNA is NM_000203.4(IDUA)-c.1205G>A (p.Trp402Ter) mutation site, or a corresponding mutation site thereof, in the genome of a patient with Hurler syndrome.

24. The arRNA according to claim 23, wherein the arRNA is an arRNA having a length charactorastic selected from the group consisting of:
55nt-c-35nt, 55nt-c-25nt, 55nt-c-24nt, 55nt-c-21nt, 55nt-c-20nt, 55nt-c-19nt, 55nt-c-18nt, 55nt-c-17nt, 55nt-c-16nt, 55nt-c-15nt, 55nt-c-14nt, 55nt-c-13nt, 55nt-c-12nt, 55nt-c-11nt, 55nt-c-10nt, 55nt-c-9nt, 50nt-c-20nt, 50nt-c-15nt, and 45nt-c-20nt;
wherein cytidine is the targeting nucleotide in the arRNA opposite to the target adenosine, and the orientation of the arRNA is 5' to 3'.

25. The arRNA according to claim 23 or 24, wherein the arRNA comprises one or more chemical modifications.

26. The arRNA according to any one of claims 23-25, wherein the chemical modification is one or more selected from the group consisting of:
2'-O-methylation modification, phosphorothioation modification in internucleotide linkage, deoxyribonucleotide substitution modification, LNA modification, and 2'-O-(2-methoxyethyl) modification.

27. The arRNA according to any one of claims 23-26, wherein the chemical modification is one or more selected from the group consisting of:
1) 2'-O-methylation modifications in the first 2, 3, 4 or 5 nucleotides;
2) 2'-O-methylation modifications in the last 2, 3, 4, or 5 nucleotides;
3) 2'-O-methylation modifications in all the cytidines except that in the targeting triplet;
4) 2'-O-(2-methoxyethyl) modifications in the first 2, 3 or 4 nucleotides;
5) 2'-O-(2-methoxyethyl) modifications in the last 2, 3 or 4 nucleotides;
6) deoxyribonucleotide substitution modifications of the first 2, 3 or 4 nucleotides;
7) deoxyribonucleotide substitution modifications of the last 2, 3 or 4 nucleotides;
8) phosphorothioation modifications in the first 1, 2, 3, 4 or 5 internucleotide linkages;
9) phosphorothioation modifications in the last 1, 2, 3, 4 or 5 internucleotide linkages;
10) phosphorothioation modifications in all internucleotide linkages except that one or more of the 5' or 3' most adjacent internucleotide linkages of each of the nucleotides in the targeting triplet are not modified by phosphorothioation; and
11) phosphorothioation modifications in internucleotide linkages at an interval of 1, 2, 3, or 4 nucleotides except that one or more of the 5' or 3' most adjacent internucleotide linkages of each of the nucleotides in the targeting triplet are not modified by phosphorothioation.

28. The arRNA according to any one of claims 23-27, wherein the arRNA comprises any chemical modification selected from the group consisting of:
CMC: the first 3 and the last 3 nucleotides in the arRNA sequence are respectively modified by 2'-O-methylation, and the first 3 and the last 3 internucleotide linkages in the arRNA sequence are respectively modified by phosphorothioation;
CM1: the first 3 and the last 3 nucleotides in the arRNA sequence are respectively modified by 2'-O-methylation, the first 3 and the last 3 internucleotide linkages in the arRNA sequence are respectively modified by phosphorothioation, and all the uridines of the sequence are modified by 2'-O-methylation;
CM2: the first 3 and the last 3 nucleotides in the arRNA sequence are respectively modified by 2'-O-methylation, and the first 3 and last 3 internucleotide linkages in the arRNA sequence are respectively modified by phosphorothioation; and the 3' most adjacent nucleotide of the targeting nucleotide is modified by 2'-O-methylation;
CM3: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 5' most adjacent nucleotide of the targeting nucleotide is modified by 2'-O-methylation;
CM4: the first 3 and the last 3 nucleotides in the arRNA sequence are respectively modified by 2'-O-methylation, and the first 3 and the last 3 internucleotide linkages in the arRNA sequence are respectively modified by phosphorothioation; and the 3' most adjacent internucleotide linkage of each of the three bases in the targeting triplet is modified by phosphorothioation;
CM6: the first 5 and the last 5 nucleotides in the arRNA sequence are respectively modified by 2'-O-methylation, and the first 5 and the last 5 internucleotide linkages in the arRNA sequence are respectively modified by phosphorothioation;
CM148: the first 4 and the last 4 nucleotides in the arRNA sequence are respectively modified by 2'-O-(2-methoxyethyl), the first 3 and the last 4 internucleotide linkages in the arRNA sequence are respectively modified by phosphorothioation, and all the uridines of the sequence are modified by 2'-O-Me;
CM149: the first 3 and the last 3 nucleotides in the arRNA sequence are respectively modified by 2'-O-(2-methoxyethyl), the first 2 and the last 3 internucleotide linkages in the arRNA sequence are respectively modified by phosphorothioation, and all the uridines of the sequence are modified by 2'-O-Me;
CM150: the first 2 and the last 2 nucleotides in the arRNA sequence are respectively modified by 2'-O-(2-methoxyethyl), the first 1 and the last 2 internucleotide linkages in the arRNA sequence are respectively modified by phosphorothioation, and all the uridines of the sequence are modified by 2'-O-Me;
CM151: the first 4 and the last 4 nucleotides in the arRNA sequence are respectively modified by LNA, the first 3 and the last 4 internucleotide linkages in the arRNA sequence are respectively modified by phosphorothioation, and all the uridines of the sequence are modified by 2'-O-Me;
CM152: the first 3 and the last 3 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
CM153: the first 2 and the last 2 nucleotides in the arRNA sequence are respectively modified by LNA, the first 2 and the last 3 internucleotide linkages in the arRNA sequence are respectively modified by phosphorothioation, and all the uridines of the sequence are modified by 2'-O-Me;
CM94: the first 3 and the last 3 nucleotides in the arRNA sequence, all the uridines and all the cytidines except that in the targeting triplet in the arRNA sequence are modified by 2'-O-methylation; the first 4 and the last 3 internucleotide linkages in the arRNA sequence are all modified by phosphorothioation; from the 4th internucleotide linkage from the 5' end to the 3rd internucleotide linkage from the 3' end, every other internucleotide linkage is modified by phosphorothioation, except that the 5' most adjacent internucleotide linkage of each nucleotide in the targeting triple is not modified by phosphorothioation;
CM119: the first 3 and the last 3 nucleotides in the arRNA sequence, all the uridines and all the cytidines in the arRNA sequence except that in the targeting triplet, are modified by 2'-O-methylation; the first 4 and the last 3 internucleotide linkages in the arRNA sequence are all modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and the targeting nucleotide is substituted by a deoxyribonucleotide;
CM120: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages in the arRNA sequence are modified by phosphorothioation; from the 4th internucleotide linkage from the 5' end to the 5' most adjacent internucleotide linkage of the targeting nucleotide, and from the 3rd internucleotide linkage from the 3' end to the 3' most adjacent internucleotide linkage of the targeting nucleotide, every other internucleotide linkage is modified by phosphorothioation; and the targeting nucleotide is substituted by a deoxyribonucleotide;
CM123: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages are modified by phosphorothioation; from the 4th internucleotide linkage from the 5' end to the 3' most adjacent internucleotide linkage of the targeting nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide;
CM125: the first 3 and the last 3 nucleotides in the arRNA sequence, all the uridines and all the cytidines in the arRNA sequence except that in the targeting triplet are modified by 2'-O-methylation; the first 4 and the last 3 internucleotide linkages and the 3' most adjacent nucleotide linkages of the targeting nucleotide are modified by phosphorothioation; from the 4th internucleotide linkage from the 5' end to the 5' most adjacent nucleotide of the targeting nucleotide's 5' most adjacent nucleotide, and from the 3rd internucleotide linkage from the 3' end to the 3' most adjacent nucleotide of the targeting nucleotide, every other internucleotide linkage is modified by phosphorothioation; and each nucleotide in the targeting triplet is substituted by a deoxyribonucleotide.

29. The arRNA according to any one of claims 23-28, wherein the arRNA comprises any sequence selected from the group consisting of: 
1)Gm*Am*Cm*G*Cm-Cm*Cm-A*Cm-Cm*G-Um*G-Um*G-G*Um-Um*G-Cm*Um-G*Um-
Cm*Cm-A*G-G*A-Cm*G-G*Um-Cm*Cm-Cm*G-G*Cm-Cm*Um-G*Cm-G*A-Cm*A-Cm*Um-U
m*Cm-G*G-Cm-C-C-A*G-A*G-Cm*Um-G*Cm-Um*Cm-Cm*Um*Cm*Am (SEQ ID NO: 31);
 
2)Gm*Am*Cm*G*Cm-Cm*Cm-A*-Cm-Cm*G-Um*G-Um*G-G*Um-Um*G-Cm*Um-G*Um
-Cm*Cm-A*G-G*A-Cm*G-G*Um-Cm*Cm-Cm*G-G*Cm-Cm*Um-G*Cm-G*-A-Cm*A-Cm*Um-
Um*Cm-G*G-Cm*Cd-Cd*Ad*-G-A*G-Cm*Um-G*Cm-Um*Cm-Cm*Um*Cm*Am (SEQ ID NO:
31);
 
3)Gm*Am*Cm*G*Cm-Cm*Cm-A*Cm-Cm*G-Um*G-Um*G-G*Um-Um*G-Cm*Um-G*Um-
Cm*Cm-A*G-G*A-Cm*-G-G*Um-Cm*Cm-Cm*G-G*Cm-Cm*Um-G*-Cm-G*A-Cm*A-Cm*Um-
Um*Cm-G*G-Cm-Cd-Cd*Ad*G-A*G-Cm*Um-G*Cm-Um*Cm-Cm*Um*Cm*Am (SEQ ID NO:
31);
 
4)Gmoe*Amoe*Cmoe*Gmoe-C-C-C-A-C-C-G-Um-G-Um-G-G-Um-Um-G-C-Um-G-Um-C-C
-A-G-G-A-C-G-G-Um-C-C-C-G-G-C-C-Um-GC-G-A-C-A-C-Um-Um-C-G-G-C-C-A-G-A-G-C-
Um-G-C-Um-C*Cmoe*Umoe*Cmoe*Amoe (SEQ ID NO: 31); or
 
5)G+*A+*C+*G+C-C-C-A-C-C-G-Um-G-Um-G-G-Um-Um-G-C-Um-G-Um-C-C-A-G-G-A-
C-G-G-Um-C-C-C-G-G-C-C-Um-GC-G-A-C-A-C-Um-Um-C-G-G-C-C-C-A-G-A-G-C-Um-G-C-U
m-C*C+*U+*C+*A+ (SEQ ID NO: 31);
 wherein * indicates a phosphorothioation modification, m indicates a 2'-O-methylation modification, moe indicates a 2'-O-(2-methoxyethyl) modification, + indicates an LNA modification, and d indicates a deoxyribonucleotide substitution modification.

30. A construct comprising the coding sequence of the arRNA according to claim 23 or 24.

31. A composition, liposome, exosome, lipid-nanoparticle, cell, library, or kit comprising the arRNA according to any one of claims 23-29 or the construct according to claim 30.
